(19) Europäisches Patentamt
European Patent Office
Office européen des brevets

(11) EP 3 853 237 B1

(12) **EUROPEAN PATENT SPECIFICATION**

(45) Date of publication and mention of the grant of the patent:
**24.04.2024 Bulletin 2024/17**

(21) Application number: **19828850.8**

(22) Date of filing: **20.09.2019**

(51) International Patent Classification (IPC):
***C07F 9/572*** *(2006.01)* ***C07F 9/6558*** *(2006.01)*
***G01N 33/68*** *(2006.01)* ***C07K 1/107*** *(2006.01)*

(52) Cooperative Patent Classification (CPC):
**C07F 9/572; C07F 9/65583; C07K 1/1077; G01N 33/68; G01N 33/6845; G01N 33/6848**

(86) International application number:
**PCT/NL2019/050632**

(87) International publication number:
**WO 2020/060412 (26.03.2020 Gazette 2020/13)**

# (54) PHOSPHONATE-CONTAINING CROSSLINKERS

PHOSPHONATHALTIGE VERNETZER

AGENTS DE RÉTICULATION CONTENANT DU PHOSPHONATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2018 EP 18195721**

(43) Date of publication of application:
**28.07.2021 Bulletin 2021/30**

(73) Proprietor: **Universiteit Utrecht Holding B.V.**
**3584 CS Utrecht (NL)**

(72) Inventors:
- **SCHELTEMA, Richard Alexander**
  **3584 CM Utrecht (NL)**
- **STEIGENBERGER, Barbara Agnes**
  **3584 CM Utrecht (NL)**
- **HECK, Albert John Roeland**
  **3584 CM Utrecht (NL)**
- **PIETERS, Roelof Jan**
  **3584 CM Utrecht (NL)**

(74) Representative: **V.O.**
**P.O. Box 87930**
**2508 DH Den Haag (NL)**

(56) References cited:
**EP-A2- 0 481 370** **US-B2- 8 642 744**

- **MARTA, MAURIZIO ET AL.: "Bovine hemoglobin cross-linked through the .beta. chains. Functional and structural aspects", JOURNAL OF BIOLOGICAL CHEMISTRY, vol. 271, no. 13, 29 March 1996 (1996-03-29), pages 7473-7478, XP002788619, ISSN: 0021-9258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 853 237 B1

## Description

FIELD OF THE INVENTION

The invention disclosed herein relates to crosslinkers comprising a phosphonate moiety and methods for preparing or using same.

BACKGROUND

In recent years, the mass spectrometry (MS) driven structural proteomics toolkit has gained a large amount of traction with novel techniques such as crosslinking mass spectrometry (XL-MS), which *inter alia* generates distance information between interacting proteins. The technique uses small bi-functional reagents that covalently connect functional groups from amino acids or nucleotides that are in close proximity. To achieve the covalent bond, different types of chemistry can be used, for example specific chemistry to capture the side chains of lysine residues that are part of a protein. Typically, a spacer separates the reactive groups within the crosslinker and as such, the crosslinking reagent acts as a molecular ruler between the captured functional groups of the target molecule (generally proteins, peptides and polynucleotides).

Biomolecules such as proteins are typically crosslinked in their native state in solution. Then, the sample is typically alkylated, reduced and finally digested into smaller molecules (peptides in case of proteins and (oligo)nucleotides in case of polynucleotides) by a protease or a DNase. During this last step four distinct products are formed that can be identified by MS, which are below described for peptides but are equally true for (oligo)nucleotides.

The first product is linear-peptides, which consist of parts of the protein structure not captured by the reagent and provide no information about the structure.

The second product is mono-linked peptides, which consist of peptides containing amino acids that have reacted with one reactive group of the reagent while the other is quenched by *e.g.* water and as such can reveal information about the surface exposed regions of the protein.

The third product is loop-links, which consist of a single peptide with two amino acids connected by the crosslinking reagent, providing distance information within a very small area of the protein and as such is structurally informative about *e.g.* turns and loops in the protein structure.

The fourth product is intra- / inter-links, which consist of two peptides covalently bound by the crosslinking reagent and are structurally informative about the structure of a protein (intra-link) and about the interaction interface between two proteins (inter-link).

A major challenge to this technique has been the very low abundance of the inter- and intra-links compared to mostly the linear- and mono-linked peptides, making detection challenging. This is not surprising, as it is estimated from available data that the crosslink reaction efficiency is only 1-5%. Attempts to alleviate this situation have been made by extensive pre-fractionation of the peptide products with techniques like size exclusion chromatography (SEC) and/or strong cation exchange (SCX) to separate crosslinked peptide pairs from linear peptides.

These techniques use properties that are slightly exuberated for crosslinked peptides (size and charge), but which are not unique for crosslinked peptides and therefore generate large amounts of samples that still contain a high background of linear peptides. They also require large amounts of input material and measurement time.

Other attempts integrate an enrichment handle directly on the crosslinking reagent to distinguish crosslinked peptides from linear peptides.

Two distinct approaches are most commonly used here; the first uses crosslinking reagents, which are functionalized with an enrichment handle like biotin on the spacer region.

Examples hereof are described by US patent publication no. US 2012/0107855 A1, which lists, *inter alia,* biotin, streptavidin, antigens, antibodies, nucleic acid hybrids, and polyhistidines as possible affinity tags for use as enrichment handles.

Another publication describing biotin-containing crosslinkers is D. Tan et al., Trifunctional cross-linker for mapping proteinprotein interaction networks and comparing protein conformational states, eLIFE 2016, volume 5, e12509.

The downside here is that the used handles like biotin make the reagent very bulky, which sterically hinders the reagent from reaching denser regions of the protein structure. In addition, the biotin-streptavidin bond is so strong that full recovery of the molecules bound to the column in the elution step cannot always be ensured. This is also the case for all other chemical capture mechanisms described in the referenced material, e.g. the described Michael addition of a thiol or using photo crosslinking of benzophenone or others, that all have the disadvantage that covalent bonds are formed.

The second approach circumvents this problem by use of smaller functionalities on the crosslinking reagent like azides that allow performing bioorthogonal transformations after the crosslinking reaction has taken place. In this manner the crosslinked peptides can be further functionalized by e.g. a biotin-handle using 1,3-dipolar cycloadditions

(click-chemistry).

An example of an azide-containing crosslinker for use in mass spectrometry is described by R.M. Kaake et al., A new in vivo cross-linking mass spectrometry platform to define proteinprotein interactions in living cells, Mol. Cell. Proteomics 2014, volume 13, pages 3533-3543.

The downside here is that the additional functionalization step might lead to a large degree of loss in crosslinked peptides due to an incomplete functionalization and also here difficulties to achieve a complete elution of the crosslinked material from the enrichment beads are common.

An example of a phosphate-containing crosslinker is described by Marta et al., Journal of Biological Chemistry, 1996, volume 271, issue 13, pages 7473-7478. Further background references include US 8,642,744 B2, and EP 0481370 A2.

Phosphonate as an affinity tag has been used in publications of H. Huang et al., Simultaneous Enrichment of Cysteine containing Peptides and Phosphopeptides Using a Cysteine-specific Phosphonate Adaptable Tag (CysPAT) in Combination with titanium dioxide (TiO2) Chromatography, Mol. Cell. Proteomics 2016, volume 10, pages 3282-3296, and A. Boysen et al., A novel mass spectrometric strategy "REMAP" reveals Extensive O-linked protein glycosylation in Enterotoxigenic Escherichia coli, Scientific Reports, 2016, volume 6, 32016. These publications, however, do not deal with (trifunctional) crosslinkers. Hence, they do not address the problem of enrichment of inter- and/or intralinks that are only formed during crosslinking.

It is desired that compounds be developed that address one or more of the abovementioned problems, especially in the field of XL-MS.

SUMMARY OF THE INVENTION

In one aspect, the present invention pertains to compounds according to Formula (1):

Formula (1),

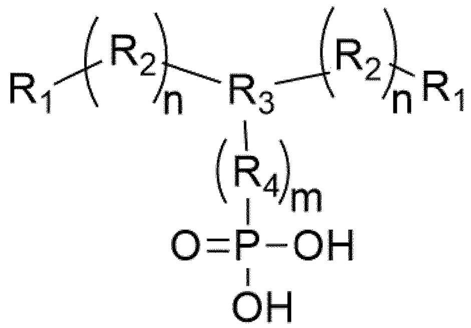

wherein m is 0 or 1,

wherein each n is independently 0 or 1,

wherein each $R_1$ is independently selected from the group consisting of - C(O)H, -C(O)Cl, -C(O)Br, -C(O)F, -C(O)-O-pentafluorophenyl, -NCS, -NCO, $-S(O)_2Cl$, $-S(O)_2F$, phenylsulfonyl fluoride, phenylsulfonyl chloride, $-C(O)-N_3$, $-C(O)OC(O)CH_3$, $-O-C(O)-O-(CH_2)_q-CH_3$, $-C(NH)-O-(CH_2)_q-CH_3$, $C_2$ epoxidyl,

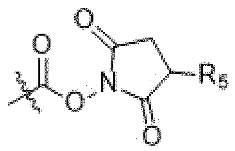 , 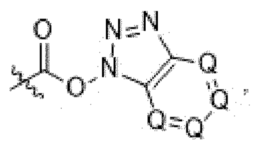 , 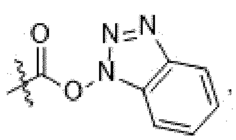 , and 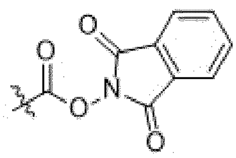 ,

wherein the wiggly lines indicate a bond to $R_2$ or to $R_3$,

wherein $R_5$ is selected from the group consisting of -H, $-CH_3$, $-CH_2CH_3$, $-O-CH_2-O-CH_3$, $-O-CH_2-O-(CH_2)_2-Si(CH_3)_3$, $SO_3H$, and $-O-CH_2-O-(CH_2)_2-O-CH_3$, wherein one Q is a nitrogen atom and the other Q are -CH-,

wherein each $R_2$ is independently selected from the group consisting of $C_{1-4}$ alkylene, $C_{2-4}$ alkenylene, $C_{2-4}$ alky-

nylene, $-S(O)-CH_2-(CH_2)_q$, $-(CH_2)_q$ $-SS-(CH_2)_q-$, $-N((CH_2)_qC(O)H)-C(O)-N((CH_2)_qC(O)H)-$, -aspartyl-prolyl-, and -valyl-prolyl-,

wherein each q is independently an integer in a range of from 0 to 6,

wherein when m is 0, then $R_3$ is selected from the group consisting of $CR_6$, benzenetriyl, 5-7 membered heteroarenetriyl, 3-7 membered (hetero)cycloalkanetriyl, 3-7 membered cycloalkenetriyl, and 5-7 membered heterocycloalkenetriyl,

wherein when m is 1, then $R_3$ is selected from the group consisting of $CR_6$, P, N, benzenetriyl, 5-7 membered heteroarenetriyl, 3-7 membered (hetero)cycloalkanetriyl, 3-7 membered cycloalkenetriyl, and 5-7 membered heterocycloalkenetriyl,

wherein the $-P(O)(OH)_2$ group is attached to a carbon atom,

wherein $R_4$ is selected from the group consisting of linear or branched $C_{1-4}$ alkylene, $C_{2-4}$ alkenylene, and $C_{2-4}$ alkynylene,

wherein $R_6$ is selected from the group consisting of hydrogen, linear or branched $C_{1-4}$ alkyl, and $C_{2-4}$ alkenyl,

wherein said benzenetriyl, heteroarenetriyl, (hetero)cycloalkanetriyl, and (hetero)cyclocalkenetriyl groups are optionally substituted with at least one group selected from the group consisting of linear or branched $C_{1-3}$ alkyl, $-O-CH_3$, -Cl, -Br, -F, and -I,

wherein said alkyl, alkenyl, alkylene, alkenylene, and alkynylene groups are optionally substituted with at least one group selected from the group consisting of =O, =NH, -Cl, -Br, -F, and -I, and optionally contain a heteroatom selected from the group consisting of N, O, and S.

In another aspect, the invention pertains to methods for preparing compounds according Formula (1), said method comprising the step of subjecting a compound according to Formula (2):

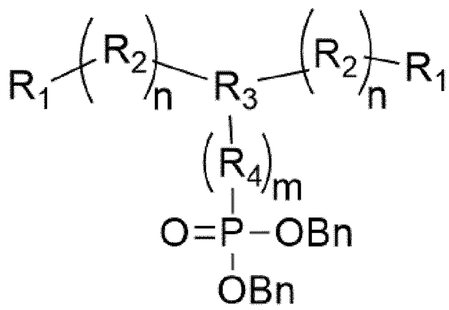

Formula (2);

to hydrogenation, wherein in Formula (2) $R_1$, $R_2$, $R_3$, $R_4$, n, and m are defined as for Formula (1).

In another aspect, the invention relates to methods for crosslinking molecules, said method comprising the step of:

a) bringing a compound according to Formula (1) into contact with at least one molecule selected from the group consisting of proteins, peptides, and polynucleotides.

In still another aspect, the invention pertains to methods for purifying crosslinked molecules, said method comprising the step of:

a) subjecting a mixture to chromatography, wherein said mixture is obtained by bringing a compound according to Formula (1) into contact with at least one molecule selected from the group consisting of proteins, peptides, and polynucleotides.

In yet a further aspect, the invention relates to methods for purifying peptides, said method comprising the subsequent steps of:

a) bringing a compound according to Formula (1) into contact with a solution comprising at least one protein;

b) bringing a reducing agent into contact with the solution obtained in step a);

c) bringing an alkylating agent into contact with the solution obtained in step b);

d) bringing a digesting agent into contact with the solution obtained in step c);

e) subjecting the solution obtained in step d) to chromatography.

In still a further aspect, the invention pertains to a method for characterizing peptides, said method comprising the steps a)-e) of the method for purifying peptides, said method further comprising a step f) that is carried out after step e), wherein step f) comprises subjecting the purified compounds obtained under step e) to mass spectrometry.

BRIEF DESCRIPTION OF THE FIGURES

Figure 1 depicts a possible explanation for how the phosphonate handle leads to excellent enrichment properties. In blue, the residue of the crosslinker according to the invention is shown, and in yellow a particle within a column with an iron complex bound to it. Panel A depicts how the phosphonate strongly binds to the column in case of a crosslinked peptide. As non-modified peptides do not carry the non-endogenous phosponate moieties these will not bind and simply flow-through during the binding step. Release can however easily be effected with an elution buffer containing 1 to 10 % ammonia or other types of buffers like phosphate buffers ensuring an efficient and full release of the bound material. Combined this may explain the higher enrichment for inter- and intra-links when using molecules of the invention.

Figure 2 depicts the enrichment efficiency on bovine serum albumin (XL: crosslinked peptides; Mono: mono-linked peptides; Peptide: linear peptides). (a) Not performing enrichment for crosslinked peptides complicates identification of crosslinked products, resulting in low identification rates for crosslinked peptides given their very low abundances compared to those of linear peptides. (b) Measuring the flow-through results in a single identified mono-linked peptide and furthermore only linear peptides, which are dominating the abundances plot. (c) Measuring the eluate results in a drastic decrease in identification and abundance levels for linear peptides. Conversely, the number of identified crosslinked peptides increases dramatically due their elevated abundance levels in the sample.

DETAILED DESCRIPTION OF THE INVENTION

The invention, in a broad sense, is based on the judicious insight that trifunctional crosslinkers according to Formula (1) possess excellent enrichment properties for inter- and intra-links after crosslinking. This is mainly due to the use of a phosphonate group as an enrichment handle. In compounds of the invention, the $R_1$ groups are able to react with naturally occurring moieties, such as amines in lysine residues within proteins. As two $R_1$ groups are present, the compounds of the invention function as a crosslinker. The two $R_1$ groups combined with the phosphonate group ensure that the compounds of the invention bear three functional groups.

Without wishing to be bound by theory, it is believed that this improvement in enrichment of inter- and intralinks is achieved as depicted in Figure 1. The phosphonate has high reactivity towards the IMAC material, while linear peptides do not and will flow-through. Release can easily and highly effectively be effected with a buffer containing 1 to 10 % ammonia or other types of buffers like phosphate buffers. It is believed that in this way, a higher enrichment selectivity towards mono-, inter- and intralinks is achieved.

In addition, after enrichment a sample of high purity has been generated from which in a single measurement a large degree of information can be obtained. If more information is required, the sample can be fractionated with techniques like SCX or SEC which are currently employed for crosslinked peptide enrichment due to their property of separating linear and mono-linked peptides from crosslinked peptides. As the linear peptides have been removed in the IMAC enrichment step, the mono-linked peptides can therefore largely be separated from the crosslinked peptides.

In another aspect compounds according to Formula (1) are small molecules that are able to reach the core of large biomolecules, for example proteins. It was found that the use of a small phosphonate handle is advantageous over other, often bulky affinity tags.

In another aspect, the molecules of the invention introduce the enrichment handle in one step (i.e. in the crosslinking step). An additional step (e.g. a click reaction) through which the enrichment handle is introduced is not required and a higher yield is achieved.

Moreover, the phosphonate group enables molecules contacted with a compound according to Formula (1) to be purified using standard chromatography techniques, such as strong anion exchange chromatography and in particular immobilized-metal affinity chromatography. Thus, the molecules and consequent sample handling steps according to the invention are easily implemented in laboratories where such chromatography setups are readily available.

Furthermore, the phosphonate groups are stable during mass spectrometry analysis, especially during fragmentation. This provides a further advantage over other affinity tags such as phosphates that are labile during that analysis (see P.J. Boersma, S. Mohammed, A.J.R. Heck, Phosphopeptide fragmentation and analysis by mass spectrometry, J. Mass Spect. 2009, volume 44, pages 861-878).

In another aspect, the phosphonate group is not cleaved off by phosphatases that are typically used to remove phosphate groups from phosphorylated peptides. This dramatically improves the performance for samples with a large degree of phosphorylated peptides. Typically, these heavily phosphorylated peptides are difficult to separate from crosslinked peptides, as the phosphate groups bind to the same column as the phosphonate tag. The entire peptide mixture, comprising phosphorylated peptides, can be treated with phosphatase, selectively removing the phosphate groups that might otherwise interfere with the phosphonate groups during chromatography steps. Hence, the background signal of non-crosslinked phosphorylated peptides is heavily reduced or even completely removed.

As yet a further advantage, the molecules of the invention are readily synthesized as depicted in Scheme 1.

## Definitions

The verb "to comprise", and its conjugations, as used in this description and in the claims is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer, unless stated otherwise.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer, unless stated otherwise.

Unless stated otherwise, the compounds of the invention and/or groups thereof may be protonated or deprotonated. It will be understood that it is possible that a compound may bear multiple charges which may be of opposite sign. For example, in a compound containing an amine and a carboxylic acid, the amine may be protonated while simultaneously the carboxylic acid is deprotonated.

It is understood that a phosphonate group can be written as - $PO(OR_a)(OR_b)$ or $-PO(OH)_2$, wherein in both cases the phosphor atom P is attached to a carbon atom that is part of the remainder of the molecule, and wherein $R_a$ and $R_b$ may be alkyl or aryl groups or combinations thereof (and $R_a$ may be the same as $R_b$).

It is further understood that the $-PO(OH)_2$ group, wherein the phosphor atom P is attached to a carbon atom that is part of the remainder of the molecule, may also be referred to as a phosphonic acid.

In several formulae, groups or substituents are indicated with reference to letters such as "Q" or "X", and various (numbered) "R" groups. In addition, the number of repeating units may be referred to with a letter, e.g. q in $-(CH_2)_q-$. The definitions of these letters are to be read with reference to each formula, i.e. in different formulae these letters, each independently, can have different meanings unless indicated otherwise.

Herein, cyclic moieties may be preceded by *e.g.* 3-7 membered, 5-7 membered, and so forth. These numbers refer to the total number of atoms within the cycle. For example, benzene is a 6 membered cycle as the ring consists of six carbon atoms. As another example, tetrahydrofuran is a 5 membered cycle as the ring consists of four carbon atoms and one oxygen atom. A further example is tetrazine, which is a 6 membered cycle as the ring consists of two carbon atoms and four nitrogen atoms.

Herein, in several chemical formulae and in text reference is made to "alkyl", "heteroalkyl", "aryl", "heteroaryl", "alkenyl", "alkynyl", "alkylene", "alkenylene", "alkynylene", "arylene", "cycloalkyl", "cycloalkenyl", "cycloakynyl", "arenetriyl", and the like. The number of carbon atoms that these groups have, excluding the carbon atoms comprised in any optional substituents as defined below, can be indicated by a designation preceding such terms (e.g. "$C_1$-$C_8$ alkyl" means that said alkyl may have from 1 to 8 carbon atoms). For the avoidance of doubt, a butyl group substituted with a $-OCH_3$ group is designated as a $C_4$ alkyl, because the carbon atom in the substituent is not included in the carbon count.

Unsubstituted alkyl groups have the general formula $C_nH_{2n+1}$ and may be linear or branched. Optionally, the alkyl groups are substituted by one or more substituents further specified in this document. Examples of alkyl groups include methyl, ethyl, propyl, 2-propyl, t-butyl, 1-hexyl, 1-dodecyl, etc. In preferred embodiments, up to two heteroatoms may be consecutive, such as in for example $-CH_2-NH-OCH_3$ and $-CH_2-O-Si(CH_3)_3$. In some preferred embodiments the heteroatoms are not directly bound to one another. Examples of heteroalkyls include $-CH_2CH_2-O-CH_3$, $-CH_2CH_2-NH-CH_3$, $-CH_2CH_2-S(O)-CH_3$, $-CH-CH-O-CH_3$, $-Si(CH_3)_3$. In preferred embodiments, a $C_1$-$C_4$ alkyl contains at most 2 heteroatoms.

An alkenyl group comprises one or more carbon-carbon double bonds, and may be linear or branched. Unsubstituted alkenyl groups comprising one C-C double bond have the general formula $C_nH_{2n-1}$. Unsubstituted alkenyl groups comprising two C-C double bonds have the general formula $C_nH_{2n-3}$. An alkenyl group may comprise a terminal carbon-carbon double bond and/or an internal carbon-carbon double bond. A terminal alkenyl group is an alkenyl group wherein a carbon-carbon double bond is located at a terminal position of a carbon chain. An alkenyl group may also comprise two or more carbon-carbon double bonds. Examples of an alkenyl group include ethenyl, propenyl, isopropenyl, t-butenyl, 1,3-butadienyl, 1,3-pentadienyl, etc.

An alkynyl group comprises one or more carbon-carbon triple bonds, and may be linear or branched. Unsubstituted alkynyl groups comprising one C-C triple bond have the general formula $C_nH_{2n-3}$. An alkynyl group may comprise a terminal carbon-carbon triple bond and/or an internal carbon-carbon triple bond. A terminal alkynyl group is an alkynyl group wherein a carbon-carbon triple bond is located at a terminal position of a carbon chain. An alkynyl group may also comprise two or more carbon-carbon triple bonds. Unless stated otherwise, an alkynyl group may optionally be substituted with one or more, independently selected, substituents as defined below. Examples of an alkynyl group include ethynyl, propynyl, isopropynyl, t-butynyl, etc.

An aryl group refers to an aromatic hydrocarbon ring system that comprises six to twenty-four carbon atoms, more preferably six to twelve carbon atoms, and may include monocyclic and polycyclic structures. When the aryl group is a polycyclic structure, it is preferably a bicyclic structure. Optionally, the aryl group may be substituted by one or more substituents further specified in this document. Examples of aryl groups are phenyl and naphthyl.

Preferably, heteroaryl groups comprise five to sixteen carbon atoms and contain between one to five heteroatoms. Preferably, heteroaryl groups comprise at least two carbon atoms (i.e. at least $C_2$) and one or more heteroatoms N, O, or S. A heteroaryl group may have a monocyclic or a bicyclic structure. Optionally, the heteroaryl group may be substituted by one or more substituents further specified in this document. Examples of suitable heteroaryl groups include pyridinyl, quinolinyl, pyrimidinyl, pyrazinyl, pyrazolyl, imidazolyl, thiazolyl, pyrrolyl, furanyl, triazolyl, benzofuranyl, indolyl, purinyl, benzoxazolyl, thienyl, phospholyl and oxazolyl.

Herein, the prefix hetero- denotes that the group contains one or more heteroatoms selected from the group consisting of O, N, and S. It will be understood that groups with the prefix hetero- by definition contain heteroatoms. Hence, it will be understood that if a group with the prefix hetero- is part of a list of groups that is defined as optionally containing heteroatoms, that for the groups with the prefix hetero- it is not optional to contain heteroatoms, but is the case by definition.

When referring to a (hetero)aryl group the notation is meant to include an aryl group and a heteroaryl group. In general, when (hetero) is placed before a group, it refers to both the variant of the group without the prefix hetero- as well as the group with the prefix hetero-.

Herein, the prefix cyclo- denotes that groups are cyclic. An example of a cyclic group is cyclopropyl.

Herein, the suffix -ene denotes divalent groups, i.e. that the group is linked to at least two other moieties. An example of an alkylene is propylene ($-CH_2-CH_2-CH_2-$), which is linked to another moiety at both termini. It is understood that if a group with the suffix -ene is substituted at one position with -H, then this group is identical to a group without the suffix. For example, an alkylene substituted with -H is identical to an alkyl group. I.e. propylene, $-CH_2-CH_2-CH_2-$, substituted with -H at one terminus, $-CH_2-CH_2-CH_2-H$, is logically identical to propyl, $-CH_2-CH_2-CH_3$.

Herein, the suffix -triyl denotes trivalent groups, i.e. that the group is linked to at least three other moieties. An example of a benzenetriyl is depicted below:

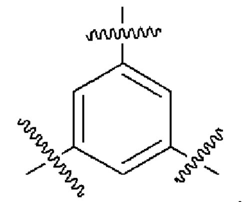

,

wherein the wiggly lines denote bonds to different groups of the main compound.

It is understood that if a group, for example an alkyl group, contains a heteroatom, then this group is identical to a hetero-variant of this group. For example, if an alkyl group contains a heteroatom, this group is identical to a heteroalkyl group. Similarly, if an aryl group contains a heteroatom, this group is identical to a heteroaryl group. It is understood that "contain" and its conjugations mean herein that when a group contains a heteroatom, this heteroatom is part of the backbone of the group. For example, a $C_2$ alkylene containing an N refers to $-NH-CH_2-CH_2-$, $-CH_2-NH-CH_2-$, and $-CH_2-CH_2-NH-$.

If indicated that a group (optionally) contains a heteroatom, the group may contain a heteroatom at non-terminal positions or at one or more terminal positions. In this case, "terminal" refers to the terminal position within the group, and not necessarily to the terminal position of the entire compound. For example, if an ethylene group contains a nitrogen atom, this may refer to $-NH-CH_2-CH_2-$, $-CH_2-NH-CH_2-$, and $-CH_2-CH_2-NH-$. For example, if an ethyl group contains a nitrogen atom, this may refer to $-NH-CH_2-CH_3$, $-CH_2-NH-CH_3$, and $-CH_2-CH_2-NH_2$.

Herein, it is understood that cyclic compounds (i.e. aryl, cycloalkyl, cycloalkenyl, etc.) are understood to be monocyclic, polycyclic or branched. It is understood that the number of carbon atoms for cyclic compounds not only refers to the number of carbon atoms in one ring, but that the carbon atoms may be comprised in multiple rings. These rings may be fused to the main ring or substituted onto the main ring.

Unless stated otherwise, any group disclosed herein that is not cyclic is understood to be linear or branched. In particular, (hetero)alkyl groups, (hetero)alkenyl groups, (hetero)alkylene groups, (hetero)alkenylene groups, (hetero)alkynylene groups, and the like are linear or branched, unless stated otherwise.

The term "protein" is herein used in its normal scientific meaning. Herein, polypeptides comprising about 10 or more amino acids are considered proteins. A protein may comprise natural, but also unnatural amino acids. The term "protein" herein is understood to comprise antibodies and antibody fragments.

The term "peptide" is herein used in its normal scientific meaning. Herein, peptides are considered to comprise a number of amino acids in a range of from 2 to 9.

The term "polynucleotide" is herein used in its normal scientific meaning. Polynucleotides may for example be deoxyribonucleic acid (DNA) or ribonucleic acid (RNA). Unless indicated otherwise, polynucleotides as disclosed herein can be single-stranded or double-stranded. Herein, polynucleotides are understood to comprise at least 13 nucleotides.

The term "activated carboxylic acid" herein refers to a carboxylic acid moiety (i.e. -COOH) that has been modified in such a way that the poor leaving group -OH has been replaced with a good leaving group. In particular, activated carboxylic acids as used herein may refer to moieties selected from the group consisting of -C(O)Cl, -C(O)Br, -C(O)F, -C(O)-O-pentafluorophenyl,

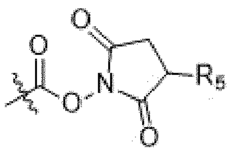 , 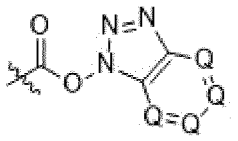 , 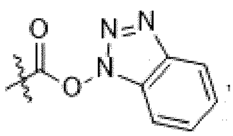 , and 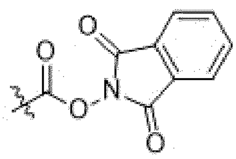 ,

wherein $R_5$ and Q are as defined for Formula (1).

The compounds of the invention may be present as a salt, unless explicitly stated otherwise.

The term "salt thereof means a compound formed when an acidic proton, typically a proton of an acid, is replaced by a cation, such as a metal cation or an organic cation and the like.

"Salt" refers to salts derived from a variety of organic and inorganic counter ions known in the art and include, for example, sodium, potassium, calcium, magnesium, ammonium, tetraalkylammonium, and the like, and when the molecule contains a basic functionality, salts of organic or inorganic acids, such as hydrochloride, hydrobromide, formate, tartrate, besylate, mesylate, acetate, maleate, oxalate, and the like.

The logarithm of the partition-coefficient, i.e. Log P, is herein used as a measure of the hydrophobicity of a compound. Typically, the Log P is defined as

$$\log\left(\frac{[Solute]_{octanol}^{un-ionized}}{[Solute]_{water}^{un-ionized}}\right)$$

The skilled person is aware of methods to determine the partition-coefficient of compounds without undue experimentation. Alternatively, the skilled person knows that software is available to reliably estimate the Log P value, for example as a function within ChemDraw® software or online available tools.

The unified atomic mass unit or Dalton is herein abbreviated to Da. The skilled person is aware that Dalton is a regular unit for molecular weight and that 1 Da is equivalent to 1 g/mol (grams per mole).

It will be understood that herein, the terms "moiety" and "group" are used interchangeably when referring to a part of a molecule.

It will be understood that when a heteroatom is denoted as $-X(R')_2-$, wherein X is the heteroatom and R' is a certain moiety, then this denotes that two moieties R' are attached to the heteroatom.

Embodiments

The compounds of the invention satisfy Formula (1):

Formula (1),

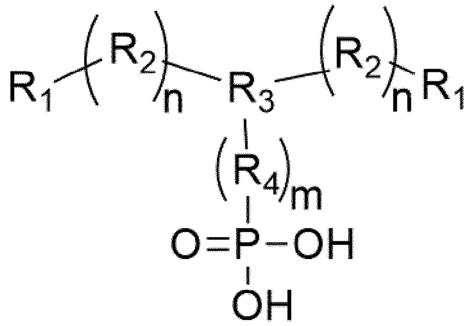

wherein $R_1$, $R_2$, $R_3$, $R_4$, m, and n are defined as disclosed herein.

In Formula (1), m is 0 or 1.

In Formula (1), each n is independently 0 or 1.

In Formula (1), the $-P(O)(OH)_2$ group is attached to a carbon atom.

In Formula (1), each $R_1$ is independently selected from the group consisting of -C(O)H, -C(O)Cl, -C(O)Br, -C(O)F, -C(O)-O-pentafluorophenyl, - NCS, -NCO, $-S(O)_2Cl$, $-S(O)_2F$, phenylsulfonyl fluoride, phenylsulfonyl chloride, $-C(O)-N_3$, $-C(O)OC(O)CH_3$, $-O-C(O)-O-(CH_2)_q-CH_3$, $-C(NH)-O-(CH_2)_q-CH_3$, $C_2$ epoxidyl,

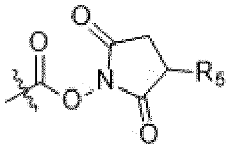 , 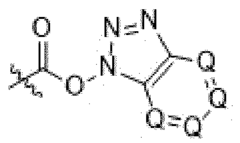 , 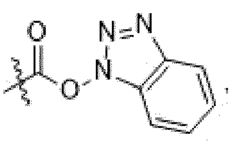 , and 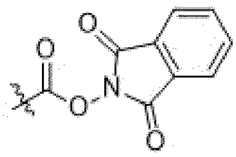 ,

wherein the wiggly lines indicate a bond to $R_2$ or to $R_3$. Preferably, both $R_1$ are -C(O)O-N-succinimide.

In Formula (1), one Q is a nitrogen atom and the other Q are -CH-.

In Formula (1), each $R_2$ is independently selected from the group consisting of $C_{1-4}$ alkylene, $C_{2-4}$ alkenylene, $C_{2-4}$ alkynylene, $-S(O)-CH_2-(CH_2)_q-$, $-(CH_2)_q-SS-(CH_2)_q-$, $-N((CH_2)_qC(O)H)-C(O)-N((CH_2)_qC(O)H)-$, -aspartyl-prolyl-, and -valyl-prolyl-.

In preferred embodiments, $R_2$ is a $C_{1-4}$ alkylene, preferably methylene, ethylene, propylene, or butylene.

In Formula (1), each q is independently an integer in a range of from 0 to 6. In some embodiments, each q is independently an integer in a range of from 0 to 5, more preferably in a range of from 0 to 4, even more preferably in a range of from 0 to 3, more preferably still in a range of from 0 to 2, most preferably each q is independently 0 or 1.

In preferred embodiments, $R_2$ is a gas-phase cleavable linker selected from the group consisting of $-S(O)-CH_2-(CH_2)_q$, $-(CH_2)_q$ $-SS-(CH_2)_q-$, $-N((CH_2)_qC(O)H)-C(O)-N((CH_2)_qC(O)H)-$, -aspartyl-prolyl-, and -valyl-prolyl-.

Gas-phase cleavable linkers are advantageous as they provide additional tools to obtain distinguishing ions when different types of gas phase fragmentation methods are used, and/or different levels of tandem mass spectrometry (such as MS2 and MS3). Thus, gas-phase cleavable linkers are able to improve the characterization of crosslinked proteins and peptides. Examples of such linkers are for instance described in A. Kao, et al, Development of a novel cross-linking strategy for fast and accurate identification of cross-linked peptides of protein complexes, Molecular and Cellular Proteomics, 2011; MQ Müller, F. Dreiocker, C.H. Ihling, M. Schäfer;,A. Sinz, Cleavable cross-linker for protein structure analysis: reliable identification of cross-linking products by tandem MS, Analytical Chemistry, 2010; and A.S. Argo, C. Shi, F. Liu, M.B. Goshe, Performing protein crosslinking using gas-phase cleavable chemical crosslinkers and liquid chromatography-tandem mass spectrometry, Methods 2015, volume 89, pages 64-73.

In Formula (1), when m is 0, then $R_3$ is selected from the group consisting of $CR_6$, benzenetriyl, 5-7 membered heteroarenetriyl, 3-7 membered (hetero)cycloalkanetriyl, 3-7 membered cycloalkenetriyl, and 5-7 membered heterocycloalkenetriyl.

When m is 1, then $R_3$ is selected from the group consisting of $CR_6$, P, N, benzenetriyl, 5-7 membered heteroarenetriyl, 3-7 membered (hetero)cycloalkanetriyl, and 3-7 membered cycloalkenetriyl, and 5-7 membered heterocycloalkenetriyl.

Preferred 5-7 membered heteroarenetriyl groups for $R_3$ are pyridinetriyl, pyrimidinetriyl, triazinetriyl, pyrazinetriyl, furantriyl, pyrroletriyl, imidazoletriyl, thiophenetriyl, oxazoletriyl, thiazoletriyl, and triazoletriyl groups. Preferably, heteroarenetriyl groups for $R_3$ are 5-6 membered.

Preferred 3-7 membered cycloalkanetriyl groups for $R_3$ are cyclopropanetriyl, cyclobutanetriyl, cyclopentanetriyl, cyclohexanetriyl, and cycloheptanetriyl groups.

Preferred 3-7 membered heterocycloalkanetriyl groups for $R_3$ are aziridinetriyl, azetidinetriyl, oxetanetriyl, 1,3-oxazetidinetriyl, tetrahydrofurantriyl, pyrrolidinetriyl, imidazolidinetriyl, piperidinetriyl, piperazinetriyl, morpholinetriyl, thiomorpholinetriyl, oxathianetriyl, oxanetriyl, dioxanetriyl, thianetriyl, dithianetriyl, thiepanetriyl, oxepanetriyl, azepanetriyl, oxazepanetriyl, oxathiepanetriyl, thiazepanetriyl, dithiepanetriyl, dioxepanetriyl, and diazepanetriyl groups.

Preferred 3-7 membered cycloalkenetriyl groups for $R_3$ are cyclopropenetriyl, cyclobutenetriyl, cyclopentenetriyl, cyclopentadienetriyl, cyclohexenetriyl, cyclohexadienetriyl, cycloheptenetriyl, cycloheptadienetriyl, and cycloheptatrienetriyl groups.

Preferred 5-7 membered heterocycloalkenetriyl groups for $R_3$ are dihydrofurantriyl, dihydrothiophenetriyl, pyrrolinetriyl, tetrahydropyridinetriyl, dihydropyridinetriyl, tetrahydropyrimidinetriyl, dihydrooxazinetriyl, dihydrothiazinetriyl, dihydrothiopyrantriyl, thiopyrantriyl, dithiinetriyl, oxathiinetriyl, dihydropyrantriyl, pyrantriyl, dioxinetriyl, tetrahydroazepinetriyl, tetrahydrooxepinetriyl, tetrahydrooxazepinetriyl, tetrahydrothiazepinetriyl, tetrahydrothiepinetriyl, dihydrodioxepinetriyl, dihydrodithiepinetriyl, tetrahydrodiazepinetriyl, dihydrooxathiepinetriyl, dihydroazepinetriyl, dihydrodiazepinetriyl, dihydrotriazepinetriyl, dihydrooxazepinetriyl, dihydrothiazepinetriyl, dioxepinetriyl, dihydrooxepinetriyl, azepinetriyl, thiazepinetriyl, thiepinetriyl, dihydrothiepinetriyl, dithiepinetriyl, oxathiazepinetriyl, and oxazepinetriyl.

Regardless of whether m is 0 or 1, $R_3$ is preferably benzenetriyl or pyrroletriyl.

In Formula (1), $R_4$ is selected from the group consisting of linear or branched $C_{1-4}$ alkylene, $C_{2-4}$ alkenylene, and $C_{2-4}$ alkynylene. Preferably, $R_4$ is $C_{1-4}$ alkylene, and more preferably $R_4$ is $C_{1-2}$ alkylene (i.e. methylene or ethylene).

In Formula (1), $R_5$ is selected from the group consisting of -H, $-CH_3$, $- CH_2CH_3$, $-O-CH_2-O-CH_3$, $-O-CH_2-O-(CH_2)_2-Si(CH_3)_3$, $SO_3H$, and $-O-CH_2-O-(CH_2)_2-O-CH_3$. Preferably, $R_5$ is -H.

In Formula (1), $R_6$ is selected from the group consisting of hydrogen, linear or branched $C_{1-4}$ alkyl, and $C_{2-4}$ alkenyl. Preferably, $R_6$ is selected from the group consisting of hydrogen, methyl, and ethyl.

In Formula (1), the benzenetriyl, heteroarenetriyl, (hetero)cycloalkanetriyl, and (hetero)cyclocalkenetriyl groups are optionally substituted with at least one group selected from the group consisting of linear or branched $C_{1-3}$ alkyl, $-O-CH_3$, -Cl, -Br, -F, and -I.

Preferably, the benzenetriyl, heteroarenetriyl, (hetero)cycloalkanetriyl, and (hetero)cyclocalkenetriyl groups are optionally substituted with at most three, more preferably at most two, most preferably at most one group selected from the group consisting of linear or branched $C_{1-3}$ alkyl, $-O-CH_3$, -Cl, -Br, -F, and -I.

In Formula (1), the alkyl, alkenyl, alkylene, alkenylene, and alkynylene groups are optionally substituted with at least one group selected from the group consisting of =O, =NH, -Cl, -Br, -F, and -I, and optionally contain a heteroatom selected from the group consisting of N, O, and S.

Preferably, the alkyl, alkenyl, alkylene, alkenylene, and alkynylene groups are optionally substituted with at most three, preferably at most two, more preferably at most one group selected from the group consisting of =O, =NH, -Cl, -Br, -F, and -I.

Preferably, the alkyl, alkenyl, alkylene, alkenylene, and alkynylene groups optionally contain at most two, more preferably at most one heteroatom selected from the group consisting of N, O, and S.

The compounds according to Formula (1) preferably have a molecular weight of at most 1500 Da. In preferred embodiments, the compounds according to Formula (1) have a molecular weight of at most 1300 Da, more preferably at most 1100 Da, even more preferably at most 900 Da.

In the compounds according to Formula (1), at least one atom selected from the group consisting of $^1H$, $^{12}C$, $^{14}N$, and $^{16}O$ may be replaced with its stable isotope analogue. If $^1H$ is replaced, it is replaced with $^2H$. If $^{12}C$ is replaced, it is replaced with $^{13}C$. If $^{14}N$ is replaced, it is replaced with $^{15}N$. If $^{16}O$ is replaced, it is replaced with $^{18}O$.

Compounds according to Formula (1) preferably have a Log P value in a range of from -2 to 1. A compound with a Log P value in this range has the advantage that it is well-soluble in aqueous solutions and has improved chance to reach the hydrophobic core of the protein.

In preferred embodiments, compounds according to Formula (1) are selected from the group consisting of

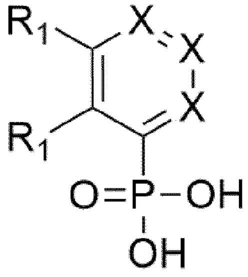
,
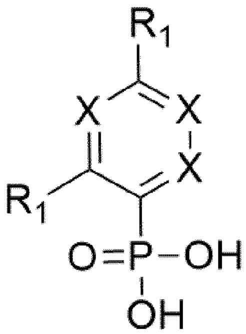
,
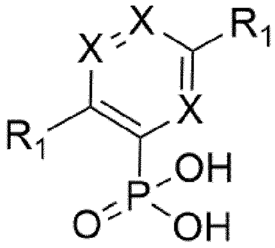
,
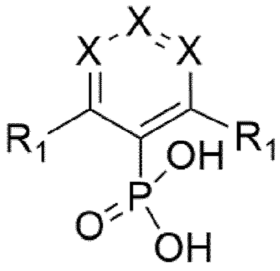
,
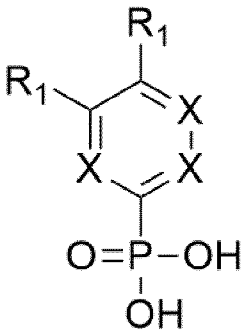
, and
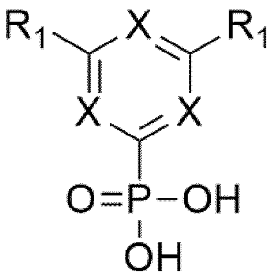
,

wherein each X is independently selected from the group consisting of $CR_6$, and N. Preferably, at most two X are N, more preferably at most one X is N. In a preferred embodiment, at least two X are CH. More preferably, all X are $CR_6$, and even more preferably all X are CH.

In other preferred embodiments, the compound according to Formula (1) is selected from the group consisting of

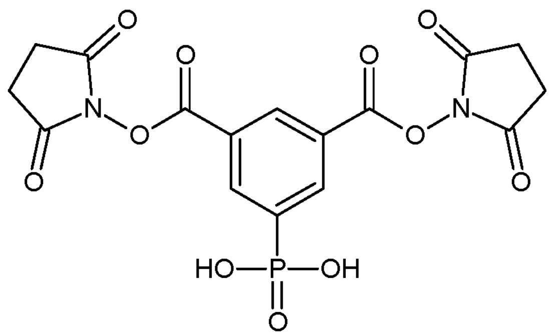
,

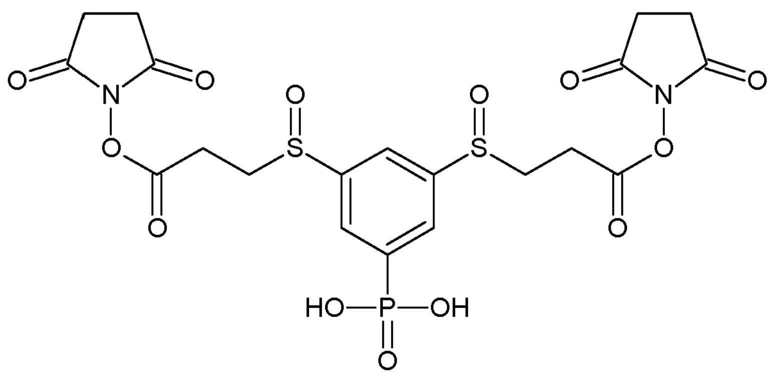

,

and

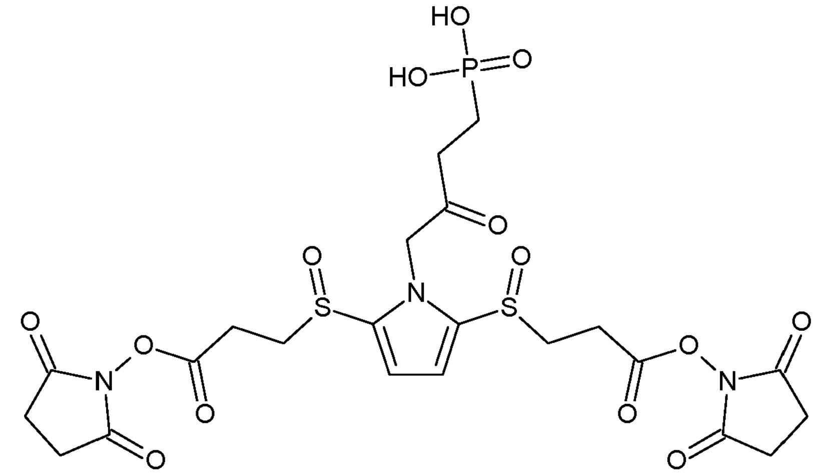

.

The invention further relates to methods for preparing compounds according to Formula (1). In a broad sense, these methods comprising the step of subjecting a compound according to Formula (2):

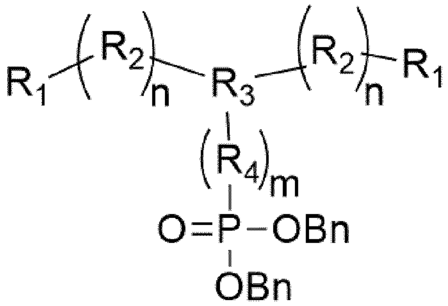

Formula (2);

to hydrogenation, wherein in Formula (2) $R_1$, $R_2$, $R_3$, $R_4$, n, and m are defined as for Formula (1).

In one embodiment, the method for preparing a compound according to Formula (1) relates to the preparation of compounds wherein each $R_1$ is independently selected from the group consisting of -C(O)Cl, -C(O)Br, - C(O)F, -C(O)-O-pentafluorophenyl,

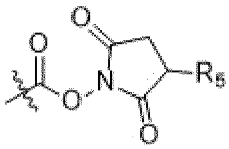 , 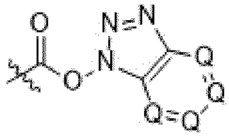 , 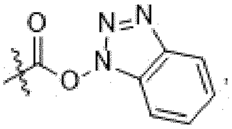 , and 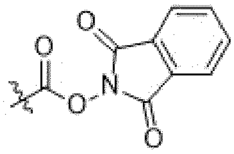 .

In this embodiment, the method comprises the subsequent steps of:

a) subjecting a compound according to Formula (3) to reduction so as to obtain a compound according to Formula (4):

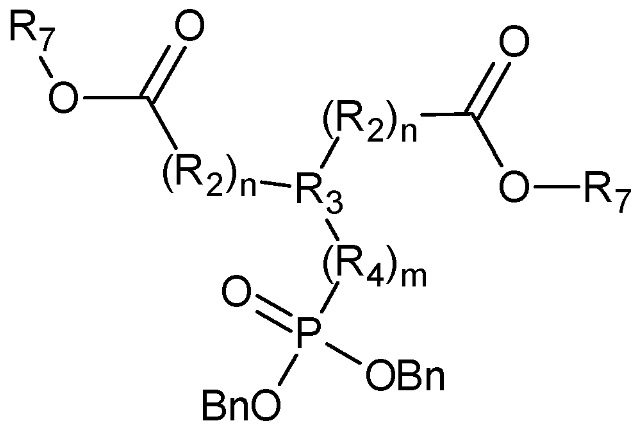

Formula (3);

wherein $R_7$ is selected from the group consisting of $-CH_3$ and $-CH_2CH_3$;

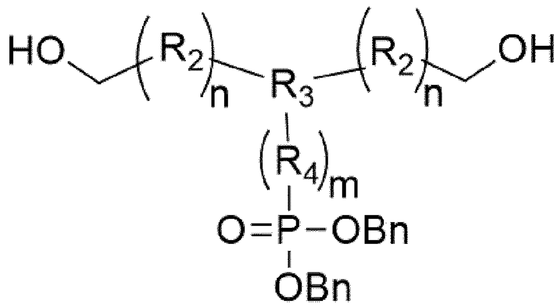

Formula (4);

b) subjecting the compound according to Formula (4) to oxidation so as to obtain a compound according to Formula (5):

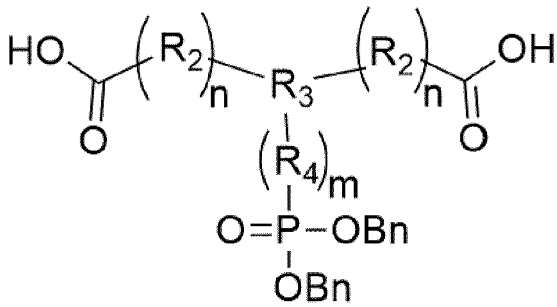

Formula (5);

c) activating the carboxylic acids of the compound according to Formula (5) so as to obtain a compound according to Formula (6):

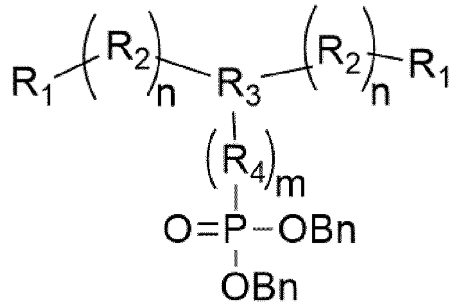

Formula (6);

d) subjecting the compound according to Formula (6) to hydrogenation. For Formulae (3) to (6) $R_2$, $R_3$, $R_4$, n, and m are defined as for Formula (1). For Formula (6) each $R_1$ is independently selected from the group consisting of -C(O)Cl, -C(O)Br, -C(O)F, -C(O)-O-pentafluorophenyl,

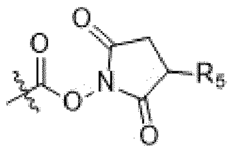 , 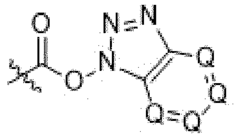 , 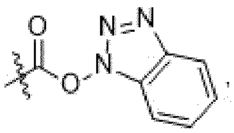 , and 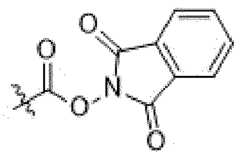

,

wherein the wiggly lines, $R_5$ and Q are as defined for Formula (1).

The methods for synthesizing compounds according to Formula (1) are based on the judicious insight to use benzyl (Bn in Formulae (2) to (6)) groups as protecting groups for the phosphonate moiety. This is advantageous, as benzyl groups are compatible with protecting groups for other moieties. Especially in the synthesis of compounds according to the invention wherein $R_1$ is an activated carboxylic acid, this strategy is particularly advantageous. It was found that in contrast with other phosphonate protecting strategies, the benzyl protection is fully compatible with the reduction/oxidation/activation strategy as described herein to synthesize the activated carboxylic acids.

An alternative synthesis route, wherein the esters in Formula (3) are first hydrolyzed proved unsuccessful. The benzyl group, like other phosphonate protecting groups, turned out to be unstable under these hydrolysis conditions. Therefore, the judicious insight to successfully synthesize compounds according to the invention by using a benzyl protecting group and the reduction/oxidation/activation strategy as described herein was surprisingly advantageous.

The invention also pertains to methods for crosslinking molecules. Methods for crosslinking molecules according to the invention comprise the step of bringing a compound according to Formula (1) into contact with at least one molecule selected from the group consisting of proteins, peptides, and polynucleotides.

Without wishing to be bound by theory, it is believed that the compounds according to Formula (1) mainly react with nucleophilic groups such as amines, thiols, and hydroxyl groups. Depending on the nature of the $R_1$ group of the compound according to Formula (1), reactivity towards specific groups can be achieved.

Furthermore, the invention is related to methods for purifying molecules crosslinked by a method of the invention. This method for purifying crosslinked molecules comprises the step of subjecting the mixture obtained by crosslinking molecules by a method of the invention to chromatography.

The invention also relates to methods for purifying peptides. These methods comprise the subsequent steps of:

a) bringing a compound according to Formula (1) into contact with a solution comprising at least one protein;
b) bringing a reducing agent into contact with the solution obtained in step a);
c) bringing an alkylating agent into contact with the solution obtained in step b);
d) bringing a digesting agent into contact with the solution obtained in step c);
e) subjecting the solution obtained in step d) to chromatography.

The skilled person is aware of suitable reducing agents, alkylating agents, and digesting agents for use in methods of the invention. Similarly, the skilled person is able to use his general knowledge about the art to select suitable buffers for the different steps of the procedures. Likewise, if required, the skilled person is aware of methods known in the art to change buffer compositions such as centrifugation, dialysis, exchange chromatography, and the like.

In preferred embodiments, any one of the steps a)-d) in the method for purifying peptides further comprises contacting the solution with a phosphatase. Most preferably, the solution obtained in step d) is contacted with a phosphatase in a separate step d2 before proceeding to step e), amounting to a method comprising the steps of

a) bringing a compound according to Formula (1) into contact with a solution comprising at least one protein;
b) bringing a reducing agent into contact with the solution obtained in step a);
c) bringing an alkylating agent into contact with the solution obtained in step b);
d) bringing a digesting agent into contact with the solution obtained in step c);
d2) bringing a phosphatase into contact with the solution obtained in step d)
e) subjecting the solution obtained in step d2) to chromatography.

Preferably, the reducing agent is selected from the group consisting of tris-(2-carboxyethyl)phosphine, thiol-containing compounds, and combinations thereof. Preferred thiol-containing compounds are selected from the group consisting of dithiothreitol, 2-mercaptoethanol, 2-mercaptoethylamine, and cysteine.

Preferably, the alkylating agent is selected from the group consisting of iodoacetamide, chloroacetamide, $C_{2-11}$ alkanal, and combinations thereof. Preferably, the alkylating agent is iodoacetamide.

Digestion of peptides and proteins can be performed using proteases or chemical agents known to the skilled person.

Preferably, digesting agents are proteases selected from the group consisting of Arg-C proteinase, Asp-N endopeptidase, caspase 1, caspase 2, caspase 3, caspase 4, caspase 5, caspase 6, caspase 7, caspase 8, caspase 9, caspase 10, chymotrypsin, clostripain, enterokinase, coagulation factor Xa, glutamyl endopeptidase, granzyme B, lysyl endopeptidase, peptidyl-Lys metalloendopeptidase, neutrophil elastase, pepsin, proline endopeptidase, proteinase K, staphylococcal peptidase I, tobacco etch virus protease, thermolysin, thrombin, trypsin, and combinations thereof. A preferred protease is trypsin. The skilled person is aware of the conditions under which these proteases function.

For the digestion of polynucleotides, preferably deoxyribonucleases (DNases) or ribonucleases (RNases) are employed. These can be either exonucleases (e.g. any one of exonucleases I, II, III, IV, V, VI, VII, or VIII) or endonucleases (e.g. DNase I).

Alternatively, digestion is performed by contacting the mixture with a chemical agent. Preferred chemical agents for digestion are selected from the group consisting of formic acid, 2-(2-nitrophenylsulfenyl)-3-methyl-3'-bromoindolenine (BNPS-skatole), cyanogen bromide, hydroxylamine, iodosobenzoic acid, and 2-nitro-5-thiocyanatobenzoic acid. The preferred chemical agent for digestion is formic acid. When applying the chemical agent for digestion, in particular formic acid, the mixture is held at elevated temperatures, such as 80 °C, or the mixture is heated up in a microwave.

The invention also pertains to methods for characterizing peptides. These methods comprise the steps a)-e) as described above for the method for purifying peptides, and further comprise a step f) that is carried out after step e). Step f) comprises subjecting the purified compounds obtained under step e) to mass spectrometry.

Preferred mass spectrometry methods are those selected from the group consisting of those using high resolution mass spectrometry like, but not restricted to Orbitrap or high resolution time-of-flight (TOF) instruments. Preferably, one mass selection device like, but not restricted to quadrupole or ion trap is incorporated. Furthermore, a high efficiency peptide fragmentation device is preferably incorporated like, but not limited to collisional activation like collision-induced dissociation (CID) and higher-energy collisional dissociation (HCD), electron activation like electron-transfer dissociation (ETD) and electron-induced dissociation (EID), ultraviolet (UV) or $CO_2$ photo dissociation (PD) needs to be incorporated.

During data acquisition, two types of methods are distinguished. For non gas-phase cleavable crosslinking reagents each precursor can be isolated and fragmented prior to recording in the mass spectrometer. In the case of collisional fragmentation it is advantageous to have the option for the isolated crosslinked peptide pair to undergo multiple rounds of activation at different energies to ensure optimal sequence coverage for both peptides. For gas-phase cleavable crosslinking reagents it is advantageous to do a tandem mass spectrometry, more specifically an MS3 experiment, where the crosslinked peptide pairs are separated from each other by a fragmentation step followed by isolation and fragmentation of each of the peptides. Alternatively, the peptide sequence information can also be read out in a single or several fragmentation steps on the crosslinked peptide pair ions.

In any of the methods of the invention comprising a chromatography step, the chromatography is preferably selected from the group consisting of immobilized-metal affinity chromatography and strong anion exchange chromatography.

In a preferred embodiment, the chromatography method chosen in any method of the invention comprising a chromatography step is immobilized-metal affinity chromatography.

In preferred embodiments, immobilized-metal affinity chromatography is performed using particles comprising a metal selected from the group consisting of $TiO_2$, $Ti^{4+}$, $Fe^{3+}$, $Ga^{3+}$, $Al^{3+}$, $Zr^{4+}$, and combinations thereof. In a particularly favorable embodiment, particles comprising $Ti^{4+}$ are used for immobilized-metal affinity chromatography.

In preferred embodiments, immobilized-metal affinity chromatography is performed using particles with a size in a range of from 4 pm to 20 pm. In a particularly favorable embodiment, particles with a size in a range of from 14 pm are used for strong anion exchange chromatrography.

In another embodiment, the chromatography method chosen in any method of the invention comprising a chromatography step is strong anion exchange chromatography.

In preferred embodiments, strong anion exchange chromatography is performed using particles comprising a moiety selected from the group consisting of trialkyl ammonium and trialkylbenzyl ammonium. In a particularly favorable embodiment, strong anion exchange chromatography is performed using particles comprising a moiety selected from the group consisting of trimethylbenzyl ammonium, dimethyl-2-hydroxyethylbenzyl ammonium, and dimethyl-2-hydroxyethylbenzyl ammonium particles are used.

More preferably, strong anion exchange chromatography is performed using particles comprising polystyrene. In another preferred embodiment, strong anion exchange chromatography is performed using particles comprising Amberlite™. In yet another preferred embodiment, strong anion exchange chromatography is performed using particles comprising Dowex®.

Herafter, the invention is further illustrated with non-limiting examples.

EXAMPLES

**Example 1: synthesis of a compound according to Formula (1)**

Scheme 1 depicts the general synthetic route towards compound (6), which is a compound according to the invention. It also provides a crystal structure of compound (6).

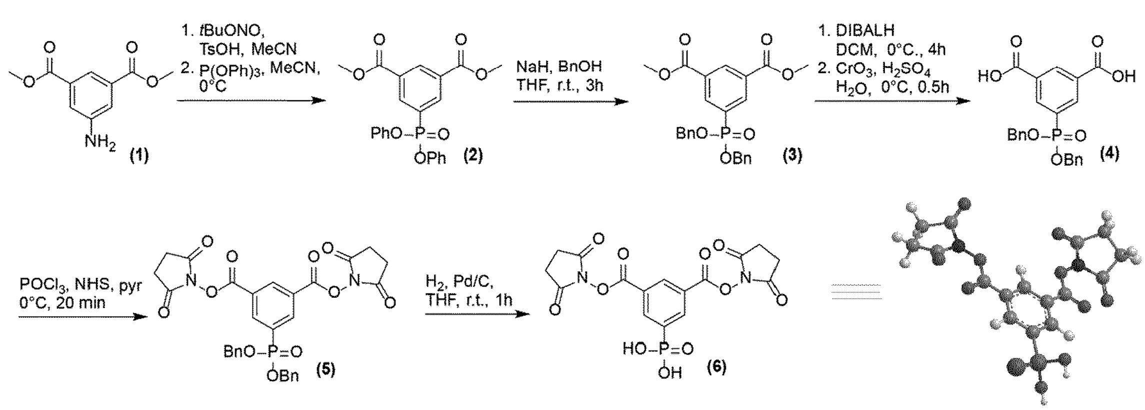


Scheme 1. Synthesis of compound (6), which is a compound according to the invention.

Below, the experimental details of each step of the synthesis in Scheme 1 are provided.

**Example 1.1: Synthesis of dimethyl 5-(diphenoxyphosphoryl)isophthalate (2)**

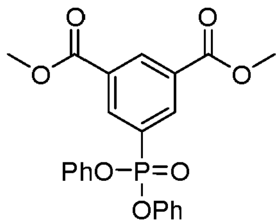

TsOH·$H_2O$ (4.4g, 22.6 mmol, 1.2eq) was dissolved in 200 mL of MeCN (HPLC grade). A slurry of Arylamine (1) (4 g, 18.9 mmol, 1eq) in 50 mL MeCN was added and a white precipitate formed. The turbid solution was cooled to 0 °C, tBuONO (3.4 mL, 28.6 mmol, 1.5eq.) was added, and the reaction mixture was stirred at 0 °C for 30 min, followed by the addition of $P(OPh)_3$ (12.6g, 57.2 mmol, 3eq). The resulting reaction solution was stirred for 8h at room temperature. During this time the reaction mixture became a clear orange-reddish solution. The solution was then concentrated under reduced pressure, and the crude residue was purified by silica gel column chromatography (PE/EtOAc 6:1) to afford product (2) (5.6 g, 13.2 mmol, 70%) as a red to pink solid.

HRMS (m/z): $[M + H]^+$ calcd. for $C_{22}H_{20}O_7P$, 427.0941; found, 427.0967 $^1$H NMR (400 MHz, $CDCl_3$) $\delta$ 8.89 (d, *J* = 1.3 Hz, 1H, Ar-*H*), 8.83 (d, *J* = 1.6 Hz, 1H, Ar-*H*), 8.79 (d, *J* = 1.6 Hz, 1H, Ar-*H*), 7.37 - 7.27 (m, *J* = 18.1, 9.8 Hz, 5H, Ph- *H*), 7.23 - 7.13 (m, 5H, Ph- *H*), 3.97 (s, 6H, 2×C$H_3$). $^{31}$**P NMR** (162 MHz, $CDCl_3$) $\delta$ 8.48 (s).

**Example 1.2: Synthesis of dimethyl 5-(bis(benzyloxy)phosphoryl)isophthalate (3)**

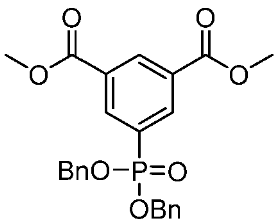

Compound (2) (899mg, 2.10 mmol) was dissolved in dry THF and the solution was cooled to 0 °C. BnOH (479μL, 4.66 mmol, 2.2eq) and NaH (199mg, 8.32mmol, 4eq.) was added and the reaction mixture was stirred for 3 h at 0 °C. After stopping the reaction by the addition on MeOH, the reaction mixture was diluted with DCM and washed with brine (2x50 mL). The organic layer was dried over $Na_2SO_4$ and the solvent was removed *in vacuo.* The crude product was purified by column chromatography (PE/EtOAc 4:1 to 2:1) to obtain (3) as a colorless oil (400mg, 0.880 mmol, 42%).

**HRMS** (m/z): $[M + Na]^+$ calcd. for $C_{24}H_{23}O_7PNa$, 477.1097; found, 477.1103. $^1$**H NMR** (600 MHz, $CDCl_3$) $\delta$ 8.80 (s, 2H, Ar-*H*), 8.59 (d, *J*= 3.4 Hz, 1H, Ar-*H*), 8.57 (d, *J* = 1.4 Hz, 1H, Ar-*H*), 7.35 - 7.26 (m, 10H, Bn-*H*), 5.17 - 5.04 (m, 4H, 2×C$H_2$), 3.94 (s, 6H, C$H_3$).

$^{13}$**C NMR** (151 MHz, $CDCl_3$) $\delta$ 165.27 (C=O), 136.84, 136.77 (C-P), 135.70 (CAr), 135.66 (*C*-Ar), 134.29 (*C*-Ar), 134.3 (*C*-Ar), 131.14(*C*-Ar), 131.04(*C*-Ar), 128.67 (*C*-Ar), 128.23 (*C*-Ar), 68.28, 68.24 (*C*$H_2$Ar), 52.7 (*C*$H_3$) $^{31}$**P NMR** (162 MHz, $CDCl_3$) $\delta$ 16.65 (s).

**Example 1.3: Synthesis of 5-(Bis(benzyloxy)phosphoryl)isophthalic acid (4a)**

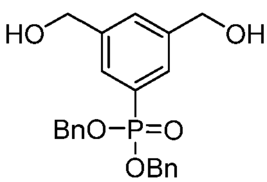

Compound (3) (390mg, 0.859 mmol) was dissolved in DCM and the solution was cooled to 0 °C. DIBALH (1M in THF, 3.43mL, 3.43 mmol, 4eq.) was added dropwise and the reaction mixture was stirred at 0 °C until TLC showed complete conversion of the starting material. The reaction was stopped by addition of MeOH. The crude reaction mixture was diluted by DCM (50 mL) and washed with brine (2x50 mL). After purification by column chromatography compound (4a) was obtained as a colourless oil (171 mg, 0.43 mmol, 50%).

**HRMS** (m/z): $[M + Na]^+$ calcd. for $C_{22}H_{23}O_5PNa$, 421.1181; found, 421.1200. $^1$**H NMR** (600 MHz, $CDCl_3$) $\delta$ 7.68 (s, 1H, Ar-*H*), 7.64 (s, 1H, Ar-*H*), 7.56 (s, 1H, Ar-*H*), 7.32 (s, 10H, Bn-*H*), 5.12 - 5.01 (m, 4H, 2×C$H_2$), 4.69 (s, 4H, 2×C$H_2$).

$^{31}$**P NMR** (162 MHz, $CDCl_3$) $\delta$ 19.38(s).

**Example 1.4: Synthesis of 5-(Bis(benzyloxy)phosphoryl)isophthalic acid (4b)**

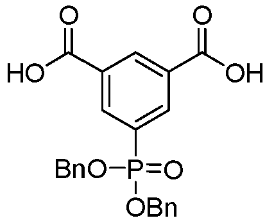

For the preparation of Jones reagent, CrOs (0.8 g, 8 mmol) was mixed with $H_2SO_4$ (0.85 mL, 16 mmol) at 0 °C. $H_2O$ (2.5 mL) was carefully added and the mixture stirred for 15 min until a dark red color remained. Alcohol (4a)

(20 mg, 0.05 mmol) was dissolved in 4 mL acetone (technical grade) and Jones reagent was added at 0 °C until the color remained red. After stirring for 3 h, TLC analysis showed complete conversion of the starting material. Isopropanol was added and the reaction color turned from green to blue. $H_2O$ was added and the aqueous phase was extracted with EtOAc, washed with brine and the combined organic phases were dried over $Na_2SO_4$, filtered and concentrated *in vacuo.* Compound (4b) was obtained without further purification was a colorless oil (21mg, 0.05mmol, quantitative). **HRMS** (m/z): $[M + Na]^+$ calcd. for $C_{22}H_{19}O_7PNa$, 449.0786; found, 449.0766. **$^1$H NMR** (600 MHz, MeOD) $\delta$ 8.75 (s, 1H, Ar-*H*), 8.49 (s, 1H, Ar-*H*), 8.45 (s, 1H, Ar-*H*), 7.33 - 7.30 (m, 10H, Bn-*H*), 5.12 - 5.01 (m, 4H, 2×C*H$_2$*). **$^{31}$P NMR** (162 MHz, MeOD) $\delta$ 19.94 (s).

**Example 1.5: Synthesis of Bis(2,5-dioxopyrrolidin-1-yl) 5-(bis(benzyloxy)phosphoryl)isophthalate (5)**

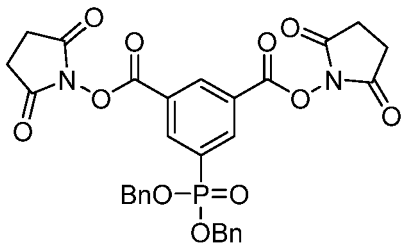

Compound (4b) (27mg, 0.063 mmol, 1eq) was dissolved in pyridine (technical grade) and NHS (20mg, 0.173 mmol, 2.5eq) was added. $POCl_3$ (15$\mu$L, 0.160 mmol, 2.7eq) was added dropwise at 0 °C and immediately a precipitate formed. The reaction mixture was stirred for 30 mins more at 0 °C and the reaction stopped by the dropwise addition of ice-cold $H_2O$. The reaction mixture was extracted with EtOAc (2x), the combined organic phases were dried over $Na_2SO_4$ and the solvent was removed *in vacuo.* The crude product was purified by column chromatography (DCM/MeOH 50:1 to 30:1) to yield NHS-ester (5) as a colourless oil (25mg, 0.040mmol, 63%).

**$^1$H NMR** (600 MHz, $CDCl_3$) $\delta$ 8.90 (s, 1H, Ar-*H*), 8.66 (dd, *J* = 13.4, 1.3 Hz, 2H, Ar-*H*), 7.32 (s, 11H, Ar-*H*), 5.19 - 5.07 (m, 5H, 2×C*H$_2$*), 2.93 (s, 8H, 4x C*H$_2$*).

**$^{31}$P NMR** (162 MHz, $CDCl_3$) $\delta$ 14.36 (s).

**Example 1.6: (3,5-Bis(((2,5-dioxopyrrolidin-1-yl)oxy)carbonyl)phenyl)phosphonic acid (6)**

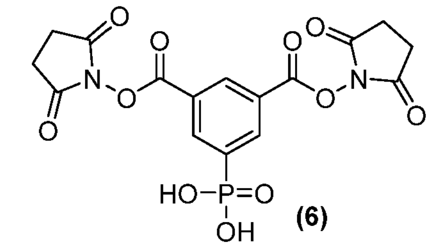

(6)

Compound (5) (11 mg, 0.017 mmol) was dissolved in dry THF. The solution was degassed for 30 min by bubbling $H_2$ through the solution. Pd/C (5mol%) was added and the reaction was set under an $H_2$-atmosphere using a balloon filled with $H_2$-gas. After stirring for 40 min at room temperature, the reaction mixture was filtered over celite and the solvent was removed *in vacuo*. The crosslinker (6) could thereby be obtained without further purification as a colorless oil (7.4 mg, 0.017mmol, quantitative).

**HRMS** (m/z): $[M + H]^+$ calcd. for $C_{16}H_{14}N_2O_{11}PNa$, 441.0330; found, 441.0390.

**$^1$H NMR** (600 MHz, MeOD) $\delta$ 8.92 (s, 1H, Ar-*H*), 8.83 (d, *J* = 12.7 Hz, 2H, 2xAr-*H*), 2.93 (s, 8H, 4×C*H*$_2$).

**$^{31}$P NMR** (162 MHz, $CDCl_3$) $\delta$ 14.36 (s).

**Example 2: crosslinking protein using a compound according to Formula (1) and digestion of the crosslinked protein**

In Scheme 2, a workflow for the crosslinking, processing and characterization of proteins is depicted. Examples 2-5 describe one or several steps of these processes.

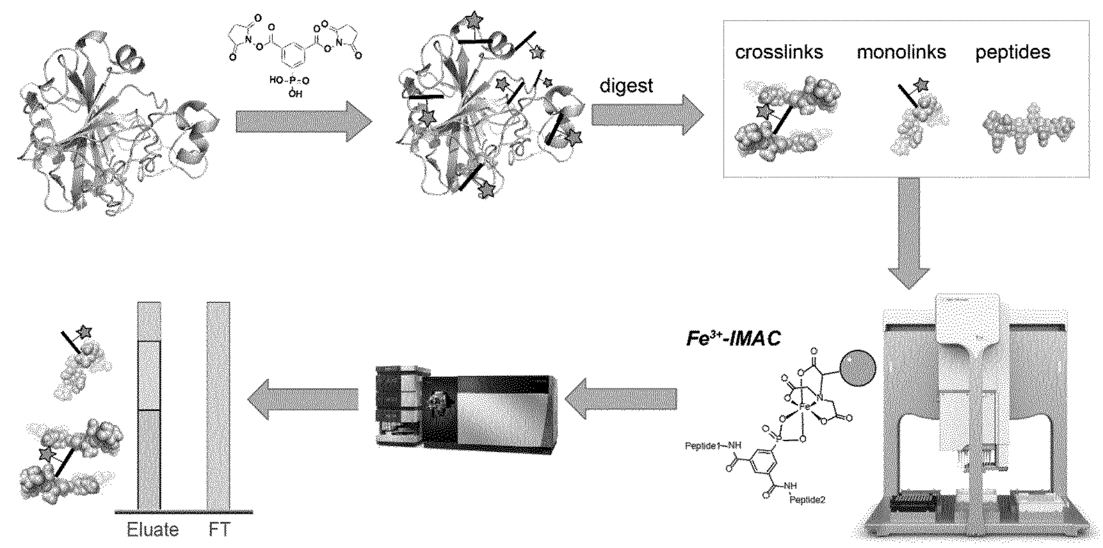


Scheme 2. Overview of a method to crosslink, digest, purify, and analyze proteins, wherein crosslinking is performed using a compound of the invention.

A crosslinking reagent according to formula (1) (i.e. compound (6)) was freshly dissolved at a concentration of 20 mM in anhydrous DMSO. The protein mixture was dissolved at a concentration of 1 mg/mL in an amine-free buffer-system. Compound (6) was added to the protein mixture at a final concentration of 2 mM and the mixture was incubated at room temperature for 45 minutes. The crosslinking reaction was quenched by addition of Tris ·HCl (100 mM, pH 8) to a final concentration of 10 mM. Residual crosslinking reagent was removed by size-cut-off filters (Vivaspin 500K 10kDa MWCO centrifugal filter units) with 3 volumes of Tris ·HCl (50 mM, pH 8). The crosslinked protein mixture (in 50 mM Tris HCl, pH 8) was reduced with DTT (final concentration of 2 mM) for 30 min at 37 °C, followed by alkylation with IAA (final concentration of 4 mM) for 30 min at 37 °C. This reaction was quenched by addition of DTT (final concentration of 2 mM). Then the sample was digested by incubation with LysC (1:75 enzyme to protein) and Trypsin (1:50 enzyme to protein) for 10 h at 37 °C, after which formic acid (1%) was added to quench the digestion. Finally, peptides were

desalted by $C_{18}$ Seppak prior to both IMAC enrichment as well as LC-MS analysis.

**Example 3: purifying peptides derived from a protein crosslinked with a compound according to Formula (1)**

Crosslinked peptides were enriched with Fe(III)-NTA 5 pL in an automated fashion using the AssayMAP Bravo Platform (Agilent Technologies; Santa Clara, Ca). Fe(III)-NTA cartridges were primed with 250 pL of 0.1% TFA in ACN and equilibrated with 250 pL of loading buffer (80% ACN/0.1% TFA). Samples were dissolved in 200 pL of loading buffer and loaded onto the cartridge. The columns were washed with 250 pL of loading buffer, and the crosslinked peptides were eluted with 25 pL of 10% ammonia directly into 25 pL of 10% formic acid. Samples were dried down and stored in 4 °C until subjected to LC-MS/MS. For LC-MS/MS analysis the samples were resuspended in 10% formic acid.

**Example 4: Mass spectrometry analysis of purified peptides crosslinked with a compound according to formula (1)**

Data were acquired using an UHPLC 1290 system (Agilent Technologies; Santa Clara, Ca) coupled on-line to an Orbitrap Fusion mass spectrometer (Thermo Scientific; San Jose, Ca). Peptides were first trapped (Dr. Maisch Reprosil C 18, 3 pm, 2 cm × 100 pm) prior to separation on an analytical column (Agilent Poroshell EC-C 18, 2.7 pm, 50 cm × 75 pm). Trapping was performed for 10 min in solvent A (0.1 M formic acid in water), and the gradient was as follows: 0 - 10% solvent B (0.1 M formic acid in 80% ACN) in 5 min, 10 - 44% in 20 min, 44 - 100% in 3 min, and finally 100% for 2 min (flow was passively split to approximately 200 nL/min). The mass spectrometer was operated in data-dependent mode. Full-scan MS spectra from m/z 350 - 1300 Th were acquired in the Orbitrap at a resolution of 60,000 after accumulation to a target value of $1 \times 10^6$ with a maximum injection time of 20 ms. In-source fragmentation was activated and set to 15 eV. The cycle time for the acquisition of MS/MS fragmentation scans was 3 s. Charge states included for MS/MS fragmentation were set to 3 - 8. Dynamic exclusion properties were set to n = 1 and to an exclusion duration of 15 s. HCD fragmentation (MS/MS) with stepped collision energy at 20, 30 and 40 NCE was performed in the Ion Trap and the spectrum acquired in the Orbitrap at a resolution of 30,000 after accumulation to a target value of $1 \times 10^5$ with an isolation window m/z 1.4 Th and maximum injection time 120 ms.

**Example 5: Data analysis of peptides crosslinked with a compound according to formula (1)**

The acquired raw data were processed using Proteome Discoverer (version 2.2.0.388) with the XlinkX nodes integrated. For linear peptides a database search was performed against a FASTA file containing only bovine serum albumin (BSA) using the standard Sequest node as the search engine. Cysteine carbamidomethylation was set as fixed modification. Methionine oxidation and protein N-term acetylation as dynamic modification. To support the search for potential monolinks, water-quenched ($C_8H_5O_6P$) and Tris-quenched ($C_{12}H_{14}O_8PN$) versions of the crosslinker according to formula (1) were additionally set as dynamic modifications. Trypsin was specified as the cleavage enzyme with a minimal peptide length of six and up to two miss cleavages were allowed. Filtering at 1 % false discovery rate (FDR) at the peptide level was applied through the Percolator node. For crosslinked peptides, a database search was performed against the same FASTA file using the XlinkX nodes for crosslink analysis. The crosslinker according to formula (1) ($C_8H_3O_5P$) was set as the crosslink modification. Cysteine carbamidomethylation was set as a fixed modification and methionine oxidation and protein N-term acetylation was set as dynamic modifications. Trypsin was specified as enzyme and up to two miss cleavages were allowed. Furthermore, identifications were only accepted with a minimal score of 40 and a minimal delta score of 4. Otherwise, standard settings were applied. Filtering at 1% FDR at peptide level was applied through the XlinkX Validator node. The standard proteome discoverer node Minora Feature Detector was used for precursor ion quantification for all identifications.

## Claims

1. A compound according to Formula (1):

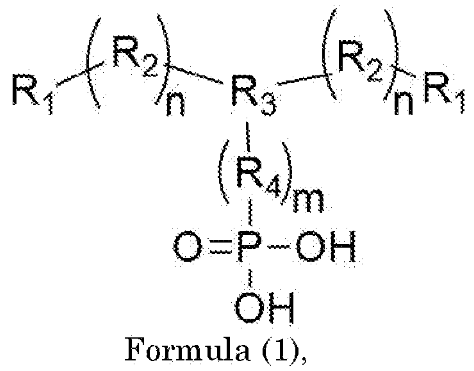

Formula (1),

wherein m is 0 or 1,

wherein each n is independently 0 or 1,

wherein each $R_1$ is independently selected from the group consisting of - C(O)H, -C(O)Cl, -C(O)Br, -C(O)F, -C(O)-O-pentafluorophenyl, -NCS, -NCO, $-S(O)_2Cl$, $-S(O)_2F$, phenylsulfonyl fluoride, phenylsulfonyl chloride, $-C(O)-N_3$, $-C(O)OC(O)CH_3$, $-O-C(O)-O-(CH_2)_n-CH_3$, $-C(NH)-O-(CH_2)_q-CH_3$, $C_2$ epoxidyl,

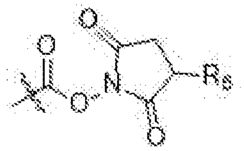 , 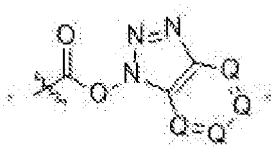 , 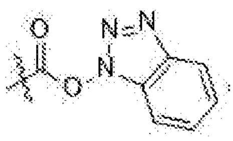 , and 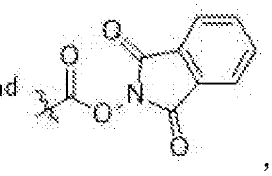 ,

wherein the wiggly lines indicate a bond to $R_2$ or to $R_3$,

wherein $R_5$ is selected from the group consisting of -H, $-CH_3$, $-CH_2CH_3$, $-O-CH_2-O-CH_3$, $-O-CH_2-O-(CH_2)_2-Si(CH_3)_3$, $-SO_3H$, and $-O-CH_2-O-(CH_2)_2-O-CH_3$, wherein one Q is a nitrogen atom and the other Q are -CH-,

wherein each $R_2$ is independently selected from the group consisting of $C_{1-4}$ alkylene, $C_{2-4}$ alkenylene, $C_{2-4}$ alkynylene, $-S(O)-CH_2-(CH_2)_q-$, $-(CH_2)_q-SS-(CH_2)_q-$, $-N((CH_2)_qC(O)H)-C(O)-N((CH_2)_qC(O)H)-$, -aspartyl-prolyl-, and -valyl-prolyl-,

wherein each q is independently an integer in a range of from 0 to 6,

wherein when m is 0, then $R_3$ is selected from the group consisting of $CR_6$, benzenetriyl, 5-7 membered heteroarenetriyl, 3-7 membered (hetero)cycloalkanetriyl, 3-7 membered cycloalkenetriyl, and 5-7 membered heterocycloalkenetriyl,

wherein when m is 1, then $R_3$ is selected from the group consisting of $CR_6$, P, N, benzenetriyl, 5-7 membered heteroarenetriyl, 3-7 membered (hetero)cycloalkanetriyl, 3-7 membered cycloalkenetriyl, and 5-7 membered heterocycloalkenetriyl,

wherein the $-P(O)(OH)_2$ group is attached to a carbon atom,

wherein $R_4$ is selected from the group consisting of linear or branched $C_{1-4}$ alkylene, $C_{2-4}$ alkenylene, and $C_{2-4}$ alkynylene,

wherein $R_6$ is selected from the group consisting of hydrogen, linear or branched $C_{1-4}$ alkyl, and $C_{2-4}$ alkenyl,

wherein said benzenetriyl, heteroarenetriyl, (hetero)cycloalkanetriyl, and (hetero)cyclocalkenetriyl groups are optionally substituted with at least one group selected from the group consisting of linear or branched $C_{1-3}$ alkyl, $-O-CH_3$, -Cl, -Br, -F, and -I,

wherein said alkyl, alkenyl, alkylene, alkenylene, and alkynylene groups are optionally substituted with at least one group selected from the group consisting of =O, =NH, -Cl, -Br, -F, and -I, and optionally contain a heteroatom selected from the group consisting of N, O, and S.

**2.** A compound according to claim 1, wherein the compound has a molecular weight of at most 1500 Da.

**3.** A compound according to any one of the preceding claims, wherein at least one atom selected from the group consisting of $^{1}H$, $^{12}C$, $^{14}N$, and $^{16}O$ is replaced with its stable isotope analogue, wherein if $^{1}H$ is replaced, it is replaced with $^{2}H$, wherein if $^{12}C$ is replaced, it is replaced with $^{13}C$, wherein if $^{14}N$ is replaced, it is replaced with $^{15}N$, and if $^{16}O$ is replaced, it is replaced with $^{18}O$.

**4.** A compound according to any one of the preceding claims, wherein both $R_1$ are -C(O)O-*N*-succinimide.

**5.** A compound according to any one of the preceding claims, wherein the compound has a Log P value in a range of from -2 to 1.

**6.** A compound according to any one of the preceding claims, wherein the compound is selected from the group consisting of

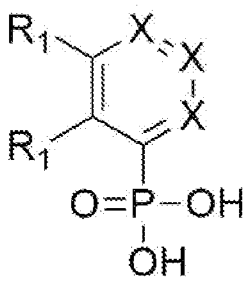 , 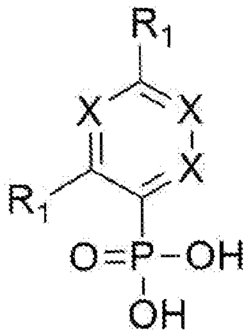 , 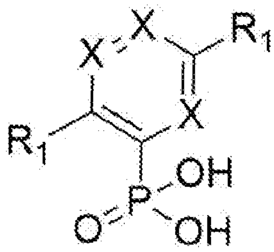 , 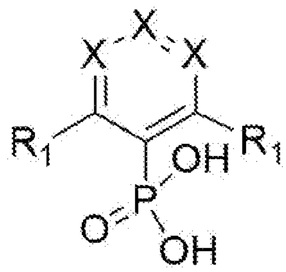 ,

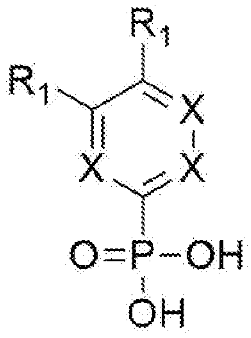 , and 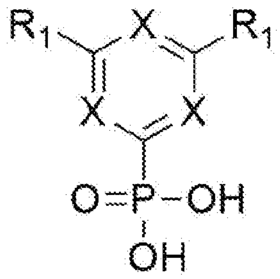 ,

wherein each X is independently selected from the group consisting of $CR_6$, and N.

**7.** A method for preparing a compound according to any one of the preceding claims, said method comprising the step of subjecting a compound according to Formula (2):

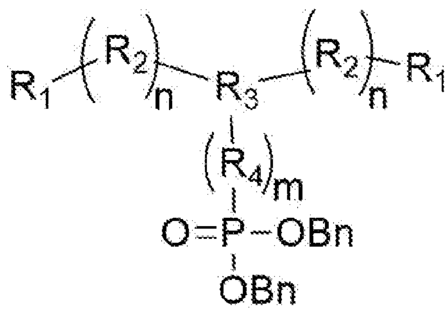

Formula (2);

to hydrogenation, wherein in Formula (2) $R_1$, $R_2$, $R_3$, $R_4$, n, and m are defined as for Formula (1).

**8.** A method for preparing a compound according to any one of claims 1 to 6, wherein in said compound each $R_1$ is independently selected from the group consisting of -C(O)Cl, -C(O)Br, -C(O)F, -C(O)-O-pentafluorophenyl,

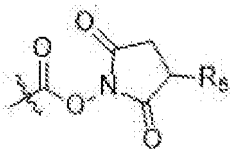 , 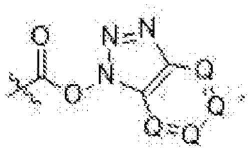 , 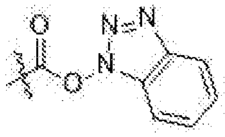 and 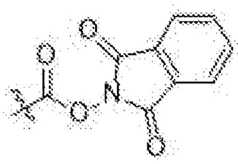 ,

wherein said method comprises the subsequent steps of:

a) subjecting a compound according to Formula (3) to reduction so as to obtain a compound according to Formula (4):

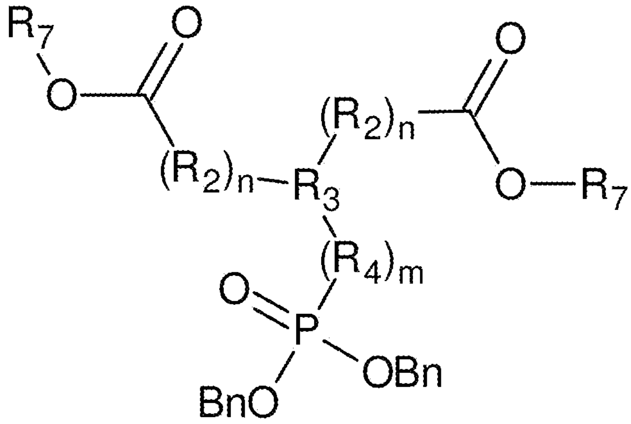

Formula (3);

wherein $R_7$ is selected from the group consisting of $-CH_3$ and $-CH_2CH_3$;

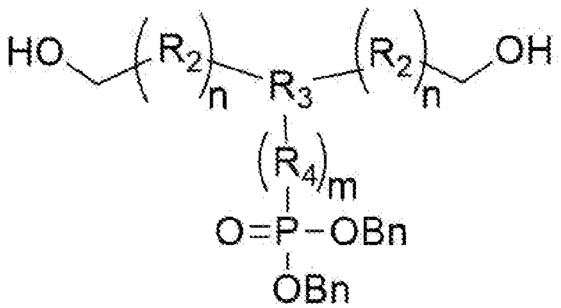

Formula (4);

b) subjecting the compound according to Formula (4) to oxidation so as to obtain a compound according to Formula (5):

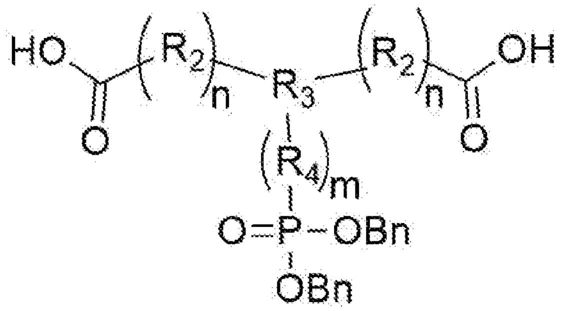

Formula (5);

c) activating the carboxylic acids of the compound according to Formula (5) so as to obtain a compound according to Formula (6):

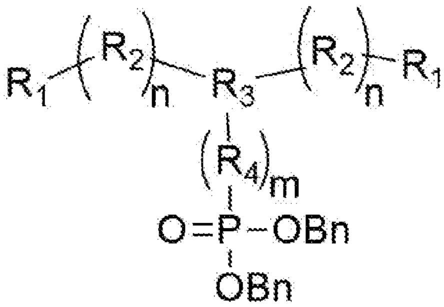

Formula (6);

d) subjecting the compound according to Formula (6) to hydrogenation, wherein for Formulae (3) to (6) $R_2$, $R_3$, $R_4$, n, and m are defined as for Formula (1), wherein for Formula (6) each $R_1$ is independently selected from the group consisting of -C(O)Cl, -C(O)Br, -C(O)F, -C(O)-O-pentafluorophenyl,

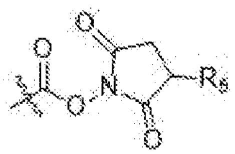 , 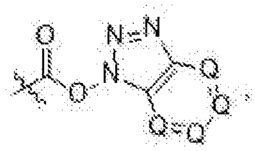 , 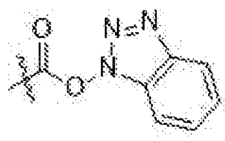 and 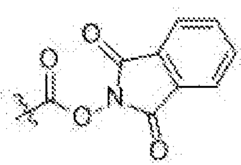 ,

wherein the wiggly lines, $R_5$ and Q are as defined for Formula (1).

**9.** A method for crosslinking molecules, said method comprising the step of:

a) bringing a compound according to any one of claims 1 to 6 into contact with at least one molecule selected from the group consisting of proteins, peptides, and polynucleotides.

**10.** A method for purifying crosslinked molecules, said method comprising the step of:

a) subjecting the mixture obtained in step a) of claim 9 to chromatography.

**11.** A method according to claim 10, wherein the chromatography is selected from the group consisting of immobilized-metal affinity chromatography and strong anion exchange chromatography.

**12.** A method for purifying peptides, said method comprising the subsequent steps of:

a) bringing a compound according to any one of claims 1 to 6 into contact with a solution comprising at least one protein;
b) bringing a reducing agent into contact with the solution obtained in step a);
c) bringing an alkylating agent into contact with the solution obtained in step b);
d) bringing a digesting agent into contact with the solution obtained in step c);
e) subjecting the solution obtained in step d) to chromatography.

**13.** A method according to claim 12, wherein the chromatography method in step e) is selected from the group consisting of immobilized-metal affinity chromatography and strong anion exchange chromatography.

**14.** A method for characterizing peptides, said method comprising the steps a)-e) according to any one of claims 12 to 13, said method further comprising a step f) that is carried out after step e), wherein step f) comprises subjecting the purified compounds obtained under step e) to mass spectrometry.

**Patentansprüche**

**1.** Verbindung gemäß der Formel (1):

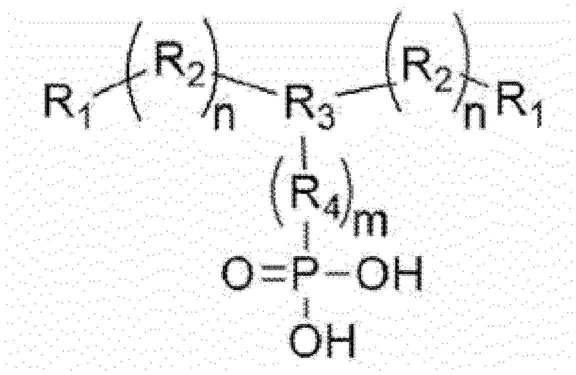

Formel (1),

wobei m gleich 0 oder 1 ist,
wobei jedes n unabhängig 0 oder 1 ist,
wobei jedes $R_1$ unabhängig ausgewählt ist aus der Gruppe bestehend aus - C(O)H, -C(O)Cl, -C(O)Br, -C(O)F, -C(O)-O-Pentafluorphenyl, -NCS, -NCO, - $S(O)_2Cl$, -$S(O)_2F$, Phenylsulfonylfluorid, Phenylsulfonylchlorid, -C(O)-$N_3$, - $C(O)OC(O)CH_3$, -O-C(O)-O-$(CH_2)_q$-$CH_3$, -C(NH)-O-$(CH_2)_q$-$CH_3$, $C_2$-Epoxidyl,

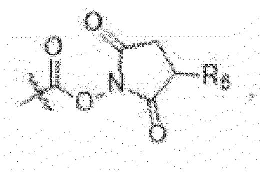 , 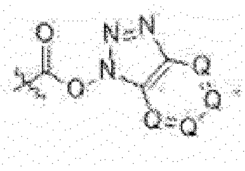 , 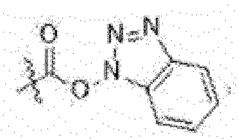 und 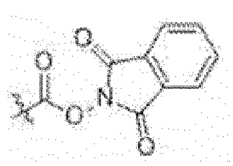

wobei die gewellten Linien eine Bindung an $R_2$ oder an $R_3$ anzeigen,
wobei $R_5$ ausgewählt ist aus der Gruppe bestehend aus - H, -$CH_3$, -$CH_2CH_3$, -O- $CH_2$-O-$CH_3$, -O-$CH_2$-O-$(CH_2)_2$-$Si(CH_3)_3$, -$SO_3H$, und -O-$CH_2$-O-$(CH_2)_2$-O-$CH_3$, wobei ein Q ein Stickstoffatom ist und die anderen Q -CH- sind, wobei jedes $R_2$ unabhängig ausgewählt ist aus der Gruppe bestehend aus $C_{1-4}$-Alkylen, $C_{2-4}$-Alkenylen, $C_{2-4}$-Alkinylen, -S(O)-$CH_2$-$(CH_2)_q$-, -$(CH_2)_q$-SS-$(CH_2)_q$-, -N($(CH_2)_q$C(O)H)-C(O)-N($(CH_2)_q$C(O)H)-, -Aspartyl-Prolyl- und Valyl-Prolyl-,
wobei jedes q unabhängig eine ganze Zahl in einem Bereich von von 0 bis 6 ist,
wobei, wenn m 0 ist, dann ist $R_3$ ausgewählt aus der Gruppe, bestehend aus $CR_6$, Benzoltriyl, 5-7-gliedrigem Heteroarentriyl, 3-7-gliedrigem (Hetero)cycloalkantriyl, 3-7-gliedrigem Cycloalkenyltriyl und 5-7-gliedrigem Heterocycloalkenyltriyl,
wobei, wenn m 1 ist, dann ist $R_3$ ausgewählt ist aus der Gruppe bestehend aus $CR_6$, P, N, Benzoltriyl, 5-7-gliedrigem Heteroarentriyl, 3-7-gliedrigem (Hetero)cycloalkantriyl, 3-7-gliedrigem Cycloalkenyltriyl und 5-7-gliedrigem Heterocycloalkenyltriyl,
wobei die $-P(O)(OH)_2$-Gruppe an ein Kohlenstoffatom angefügt ist,
wobei $R_4$ ausgewählt ist aus der Gruppe bestehend aus linearem oder verzweigtem $C_{1-4}$-Alkylen, $C_{2-4}$-Alkenylen und $C_{2-4}$-Alkinylen,
wobei $R_6$ ausgewählt ist aus der Gruppe bestehend aus Wasserstoff, linearem oder verzweigtem $C_{1-4}$-Alkyl und $C_{2-4}$-Alkenyl,
wobei die Benzoltriyl-, Heteroarentriyl-, (Hetero)cycloalkantriyl- und (Hetero)cycloalkenyltriylgruppen optional substituiert sind mit mindestens einer Gruppe, ausgewählt aus der Gruppe bestehend aus linearem oder verzweigtem $C_{1-3}$-Alkyl, -O- $CH_3$, -Cl, -Br, -F und -I,
wobei die Alkyl-, Alkenyl-, Alkylen-, Alkenylen- und Alkinylengruppen optional substituiert sind mit mindestens einer Gruppe, ausgewählt aus der Gruppe bestehend aus =O, =NH, -Cl, -Br, -F und -I, und optional ein Heteroatom enthalten, ausgewählt aus der Gruppe bestehend aus N, O und S.

**2.** Verbindung nach Anspruch 1, wobei die Verbindung ein Molekulargewicht von höchstens 1500 Da hat.

**3.** Verbindung nach einem der vorstehenden Ansprüche, wobei mindestens ein Atom, ausgewählt aus der Gruppe bestehend aus $^{1}H$, $^{12}C$, $^{14}N$, und $^{16}0$, durch sein stabiles Isotopenanalogon ersetzt ist, wobei, wenn $^{1}H$ ersetzt ist, dieses durch $^{2}H$ ersetzt ist, wobei, wenn $^{12}C$ ersetzt ist, dieses durch $^{13}C$ ersetzt ist, wobei, wenn $^{14}N$ ersetzt ist, dieses durch $^{15}N$ ersetzt ist, und wenn $^{16}O$ ersetzt ist, dieses durch $^{18}O$ ersetzt ist.

**4.** Verbindung nach einem der vorstehenden Ansprüche, wobei beide $R_1$ -C(O)O-N-Succinimid sind.

**5.** Verbindung nach einem der vorstehenden Ansprüche, wobei die Verbindung einen Log P-Wert in einem Bereich von von -2 bis 1 hat.

**6.** Verbindung nach einem der vorstehenden Ansprüche, wobei die Verbindung ausgewählt ist aus der Gruppe bestehend aus

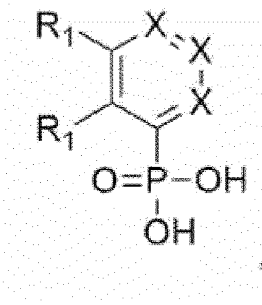,
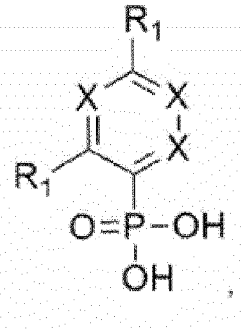,
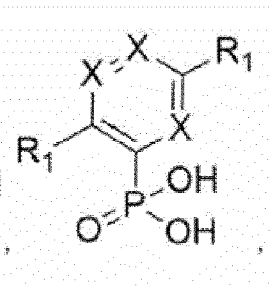,
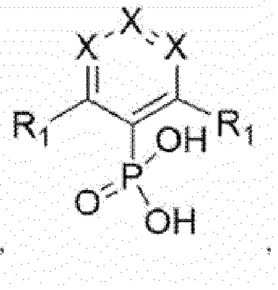,

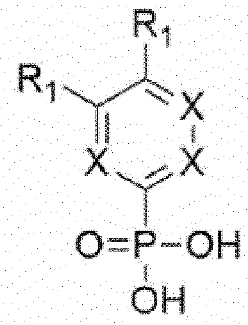, und
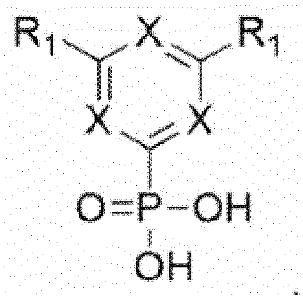.

wobei jedes X unabhängig ausgewählt ist aus der Gruppe bestehend aus CRe und N.

**7.** Verfahren zum Herstellen einer Verbindung nach einem der vorstehenden Ansprüche, das Verfahren umfassend den Schritt des Unterziehens einer Verbindung gemäß Formel (2) einer Hydrierung:

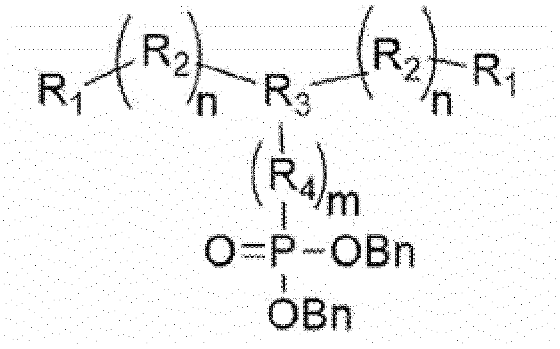

Formel (2);

wobei in Formel (2) $R_1$, $R_2$, $R_3$, $R_4$, n und m wie für Formel (1) definiert sind.

**8.** Verfahren zum Herstellen einer Verbindung nach einem der Ansprüche 1 bis 6, wobei in der Verbindung jedes $R_1$ unabhängig ausgewählt ist aus der Gruppe bestehend aus -C(O)Cl, -C(O)Br, -C(O)F, - C(O)-O-Pentafluorphenyl,

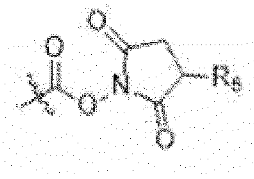 , 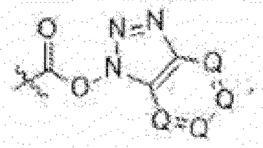 , 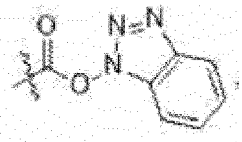 , und 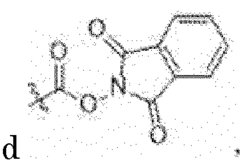 ,

wobei das Verfahren die folgenden Schritte umfasst von:

a) Unterziehen einer Verbindung gemäß Formel (3) einer Reduktion, um eine Verbindung gemäß Formel (4) zu erhalten:

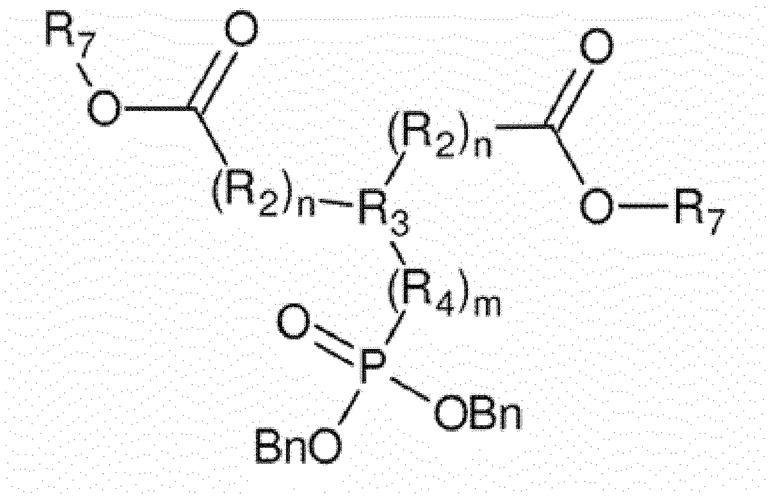

Formel (3);

wobei $R_7$ ausgewählt ist aus der Gruppe bestehend aus - $CH_3$ und $-CH_2CH_3$;

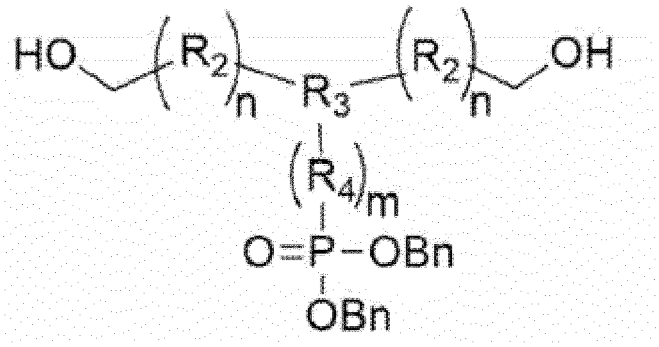

Formel (4);

b) Unterziehen der Verbindung gemäß Formel (4) einer Oxidation, um eine Verbindung gemäß Formel (5) zu erhalten:

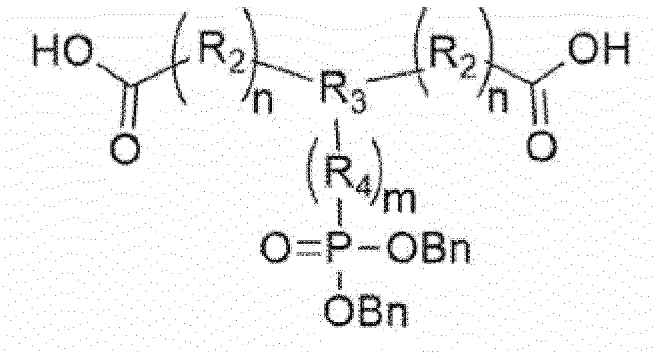

Formel (5);

c) Aktivieren der Carbonsäuren der Verbindung gemäß Formel (5), um eine Verbindung gemäß Formel (6) zu erhalten:

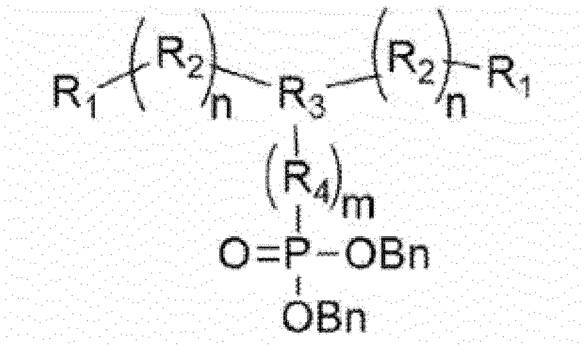

Formel (6);

d) Unterziehen der Verbindung gemäß Formel (6) einer Hydrierung, wobei für die Formeln (3) bis (6) $R_2$, $R_3$, $R_4$, n und m wie für Formel (1) definiert sind, wobei für Formel (6) jedes $R_1$ unabhängig ausgewählt ist aus der Gruppe bestehend aus -C(O)Cl, -C(O)Br, -C(O)F, -C(O)-O-Pentafluorphenyl,

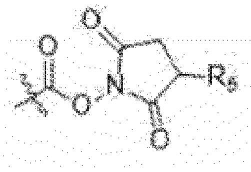, 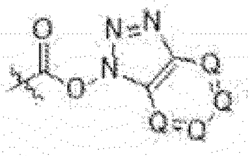, 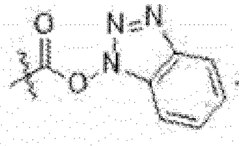, und 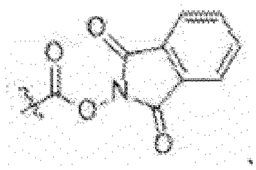.

wobei die gewellten Linien, $R_5$ und Q wie für Formel (1) definiert sind.

**9.** Verfahren zum Vernetzen von Molekülen, das Verfahren umfassend den Schritt von:

a) Inkontaktbringen einer Verbindung nach einem der Ansprüche 1 bis 6 mit mindestens einem Molekül, ausgewählt aus der Gruppe bestehend aus Proteinen, Peptiden und Polynukleotiden.

**10.** Verfahren zum Reinigen von vernetzten Molekülen, das Verfahren umfassend den Schritt von:

a) Unterziehen des Gemischs erhalten in Schritt a) von Anspruch 9 einer Chromatographie.

**11.** Verfahren nach Anspruch 10, wobei die Chromatographie ausgewählt ist aus der Gruppe bestehend aus immobilisierter Metallaffinitätschromatographie und starker Anionenaustauschchromatographie.

**12.** Verfahren zum Reinigen von Peptiden, das Verfahren umfassend die nachfolgenden Schritte von:

a) Inkontaktbringen einer Verbindung nach einem der Ansprüche 1 bis 6 mit einer Lösung, umfassend mindestens ein Protein;
b) Inkontaktbringen eines Reduktionsmittels mit der in Schritt a) erhaltenen Lösung;
c) Inkontaktbringen eines Alkylierungsmittels mit der in Schritt b) erhaltenen Lösung;
d) Inkontaktbringen eines Aufschlussmittels mit der in Schritt c) erhaltenen Lösung;
e) Unterziehen der in Schritt d) erhaltenen Lösung einer Chromatographie.

**13.** Verfahren nach Anspruch 12, wobei das Chromatographieverfahren in Schritt e) ausgewählt ist aus der Gruppe bestehend aus immobilisierter Metallaffinitätschromatographie und starker Anionenaustauschchromatographie.

**14.** Verfahren zum Charakterisieren von Peptiden, das Verfahren umfassend die Schritte a) bis e) nach einem der Ansprüche 12 bis 13, das Verfahren ferner umfassend einen Schritt f), der nach Schritt e) durchgeführt wird, wobei Schritt f) das Unterziehen der in Schritt e) erhaltenen gereinigten Verbindungen, der Massenspektrometrie umfasst.

## Revendications

**1.** Composé selon la Formule (1) :

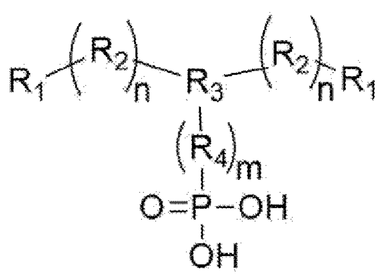

Formule (1),

dans lequel m est 0 ou 1,
dans lequel chaque n est indépendamment 0 ou 1, dans lequel chaque $R_1$ est indépendamment sélectionné dans le groupe consistant en -C(O)H, -C(O)Cl, -C(O)Br, -C(O)F, -C(O)-O-pentafluorophényle, -NCS, -NCO, -$S(O)_2Cl$, -$S(O)_2F$, fluorure de phénylsulfonyle, chlorure de phénylsulfonyle, -C(0)-$N_3$, -$C(O)OC(O)CH_3$, -O-

$C(O)-O-(CH_2)_q-CH_3$, $-C(NH)-O-(CH_2)_q-CH_3$, $C_2$ époxidyle,

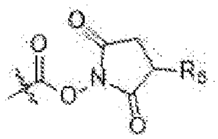, 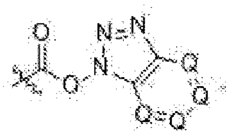, 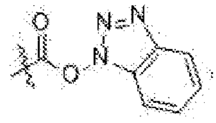 et 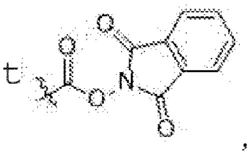,

dans lequel les lignes ondulées indiquent une liaison à $R_2$ ou à $R_3$,
dans lequel $R_5$ est sélectionné dans le groupe consistant en -H, $-CH_3$, $-CH_2CH_3$, $-O-CH_2-O-CH_3$, $-O-CH_2-O-(CH_2)_2-Si(CH_3)_3$, $-SO_3H$, et $-O-CH_2-O-(CH_2)_2-O-CH_3$, dans lequel un Q est un atome d'azote et les autres Q sont - CH-,
dans lequel chaque $R_2$ est indépendamment sélectionné dans le groupe consistant en $C_{1-4}$ alkylène, $C_{2-4}$ alcénylène, $C_{2-4}$ alcynylène, $-S(O)-CH_2-(CH_2)_q-$, $-(CH_2)_q-SS-(CH_2)_q-$, $-N((CH_2)_qC(O)H)-C(O)-N((CH_2)_qC(O)H)-$, -aspartyl-prolyl-, et -valyl-prolyl-,
dans lequel chaque q est indépendamment un entier dans une plage de 0 to 6,
dans lequel lorsque m est 0, alors $R_3$ est sélectionné dans le groupe consistant en $CR_6$, benzènetriyle, hétéroarènetriyle de 5-7 chaînons, (hétéro)cycloalkanetriyle de 3-7 chaînons, cycloalcènetriyle de 3-7 chaînons, et hétérocycloalcènetriyle de 5-7 chaînons,
dans lequel lorsque m est 1, alors $R_3$ est sélectionné dans le groupe consistant en $CR_6$, P, N, benzènetriyle, hétéroarènetriyle de 5-7 chaînons, (hétéro)cycloalkanetriyle de 3-7 chaînons, cycloalcènetriyle de 3-7 chaînons, et hétérocycloalcènetriyle de 5-7 chaînons,
dans lequel le groupe $-P-(O)(OH)_2$ est lié à un atome de carbone,
dans lequel $R_4$ est sélectionné dans le groupe consistant en $C_{1-4}$ alkylène, $C_{2-4}$ alcénylène, et $C_{2-4}$ alcynylène linéaires ou ramifiés,
dans lequel $R_6$ est sélectionné dans le groupe consistant en hydrogène, $C_{1-4}$ alkyle, et $C_{2-4}$ alcényle linéaires ou ramifiés,
dans lequel lesdits groupes benzènetriyle, hétéroarènetriyle, (hétéro)cycloalkanetriyle, et hétérocycloalcènetriyle sont éventuellement substitués par au moins un groupe sélectionné dans le groupe consistant en $C_{1-3}$ alkyle linéaire ou ramifié, $-O-CH_3$, - Cl, -Br, -F, et -I,
dans lequel lesdits groupes alkyle, alcényle, alkylène, alcénylène et alcynylène sont éventuellement substitués par au moins un groupe sélectionné dans le groupe consistant en =O, =NH, -Cl, -Br, -F et -I, et contiennent éventuellement un hétéroatome sélectionné dans le groupe consistant en N, O et S.

2. Composé selon la revendication 1, dans lequel le composé présente une masse moléculaire d'au plus 1 500 Da.

3. Composé selon l'une quelconque des revendications précédentes, dans lequel au moins un atome sélectionné dans le groupe consistant en $^{1}H$, $^{12}C$, $^{14}N$, et $^{16}O$ est remplacé par son analogue isotope stable, dans lequel si $^{1}H$ est remplacé, il est remplacé par $^{2}H$, dans lequel si $^{12}C$ est remplacé, il est remplacé par $^{13}C$, dans lequel si $^{14}N$ est remplacé, il est remplacé par $^{15}N$, et si $^{16}O$ est remplacé, il est remplacé par $^{18}O$.

4. Composé selon l'une quelconque des revendications précédentes, dans lequel les deux $R_1$ sont -C(O)O-N-succinimide.

5. Composé selon l'une quelconque des revendications précédentes, dans lequel le composé présente une valeur Log P dans une plage de -2 à 1.

6. Composé selon l'une quelconque des revendications précédentes, dans lequel le composé est sélectionné dans le groupe consistant en

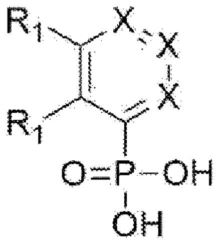 , 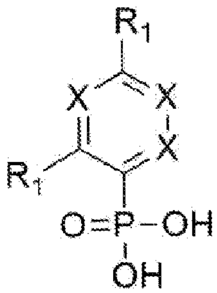 , 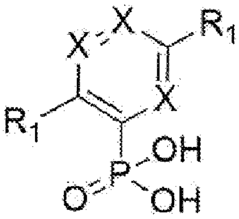 , 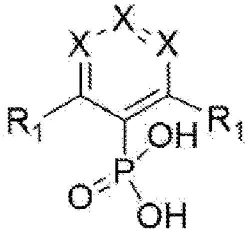 ,

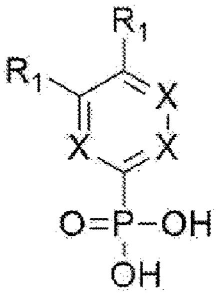 , et 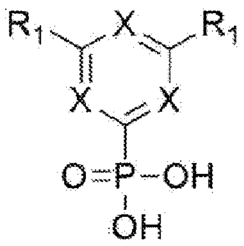 ,

dans lequel chaque X est indépendamment sélectionné dans le groupe consistant en $CR_6$, et N.

**7.** Méthode de préparation d'un composé selon l'une quelconque des revendications précédentes, ladite méthode comprenant l'étape consistant à soumettre un composé selon la Formule (2) :

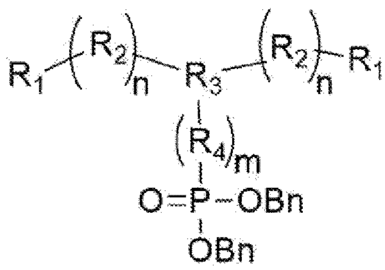

Formule (2) ;

à une hydrogénation, dans laquelle dans la Formule (2) $R_1$, $R_2$, $R_3$, $R_4$, n et m dont tels que définis pour la Formule (1).

**8.** Méthode de préparation d'un composé selon l'une quelconque des revendications 1 à 6, dans laquelle dans ledit composé chaque $R_1$ est indépendamment sélectionné dans le groupe consistant en -C(O)Cl, -C(O)Br, -C(O)F, -C(O)-O-pentafluorophényle,

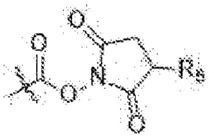 , 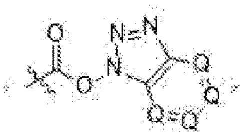 , 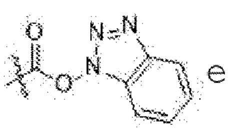 et 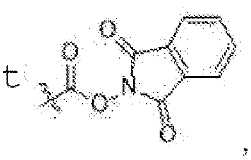 ,

dans laquelle ladite méthode comprend les étapes suivantes de :

a) soumission d'un composé selon la Formule (3) à une réduction de manière à obtenir un composé selon la Formule (4) :

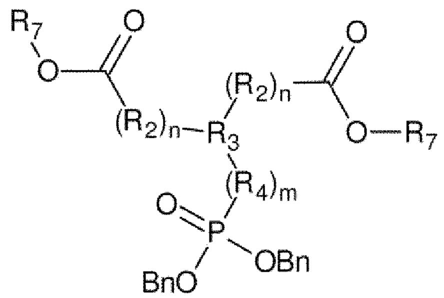

Formule (3) ;

dans laquelle $R_7$ est sélectionné dans le groupe consistant en $-CH_3$ et $-CH_2CH_3$ ;

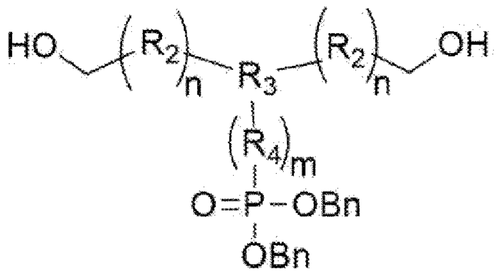

Formule (4) ;

b) soumission du composé selon la Formule (4) à une oxydation de manière à obtenir un composé selon la Formule (5) :

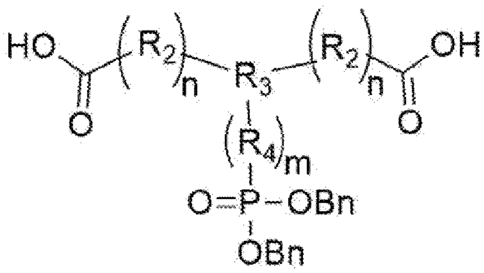

Formule (5) ;

c) activation des acides carboxyliques et du composé selon la Formule (5) de manière à obtenir un composé selon la Formule (6) :

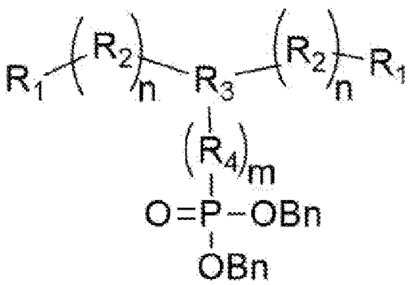

Formule (6) ;

d) soumission du composé selon la Formule (6) à une hydrogénation,

dans laquelle pour les Formules (3) à (6) $R_2$, $R_3$, $R_4$, n et m sont tels que définis dans la Formule (1), dans laquelle pour la Formule (6) chaque $R_1$ est indépendamment sélectionné dans le groupe consistant en -C(O)Cl, -C(O)Br, -C(O)F, -C(O)-O-pentafluorophényle,

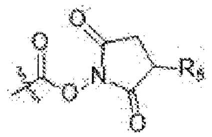, 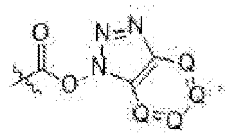 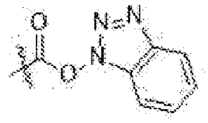 et 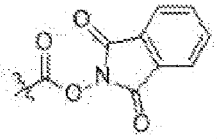 ,

dans laquelle les lignes ondulées, $R_5$ et Q sont tels que définis dans la Formule (1).

**9.** Méthode de réticulation de molécules, ladite méthode comprenant l'étape consistant à :

a) mettre en contact un composé selon l'une quelconque des revendications 1 à 6 avec au moins une molécule sélectionnée dans le groupe consistant en des protéines, des peptides, et des polynucléotides.

**10.** Méthode de purification de molécules réticulées, ladite méthode comprenant l'étape consistant à :

a) soumettre le mélange obtenu à l'étape a) de la revendication 9 à une chromatographie.

**11.** Méthode selon la revendication 10, dans laquelle la chromatographie est sélectionnée dans le groupe consistant en une chromatographie d'affinité sur ions métalliques immobilisés et une chromatographie échangeuse d'anions forts.

**12.** Méthode de purification de peptides, ladite méthode comprenant les étapes suivantes de :

a) mise en contact d'un composé selon l'une quelconque des revendications 1 à 6 avec une solution comprenant au moins une protéine ;
b) mise en contact d'un agent réducteur avec la solution obtenue à l'étape a) ;
c) mise en contact d'un agent alkylant avec la solution obtenue à l'étape b) ;
d) mise en contact d'un agent de digestion avec la solution obtenue à l'étape c) ;
e) soumission de la solution obtenue à l'étape d) à une chromatographie.

**13.** Méthode selon la revendication 12, dans laquelle la méthode de chromatographie à l'étape e) est sélectionnée dans le groupe consistant en une chromatographie d'affinité sur ions métalliques immobilisés et une chromatographie échangeuse d'anions forts.

**14.** Méthode de caractérisation de peptides, ladite méthode comprenant les étapes a)-e) selon l'une quelconque des revendications 12 à 13, ladite méthode comprenant en outre une étape f) qui est mise en œuvre après l'étape e), dans laquelle ladite étape f) comprend la soumission des composés purifiés obtenus à l'étape e) à une spectrométrie de masse.

**A.** Crosslinked peptides

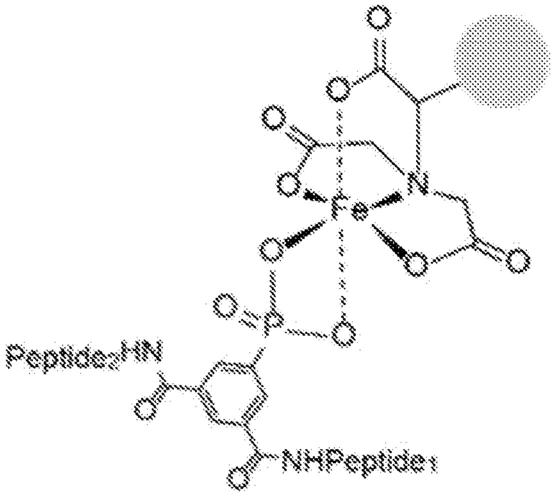

**Figure 1**

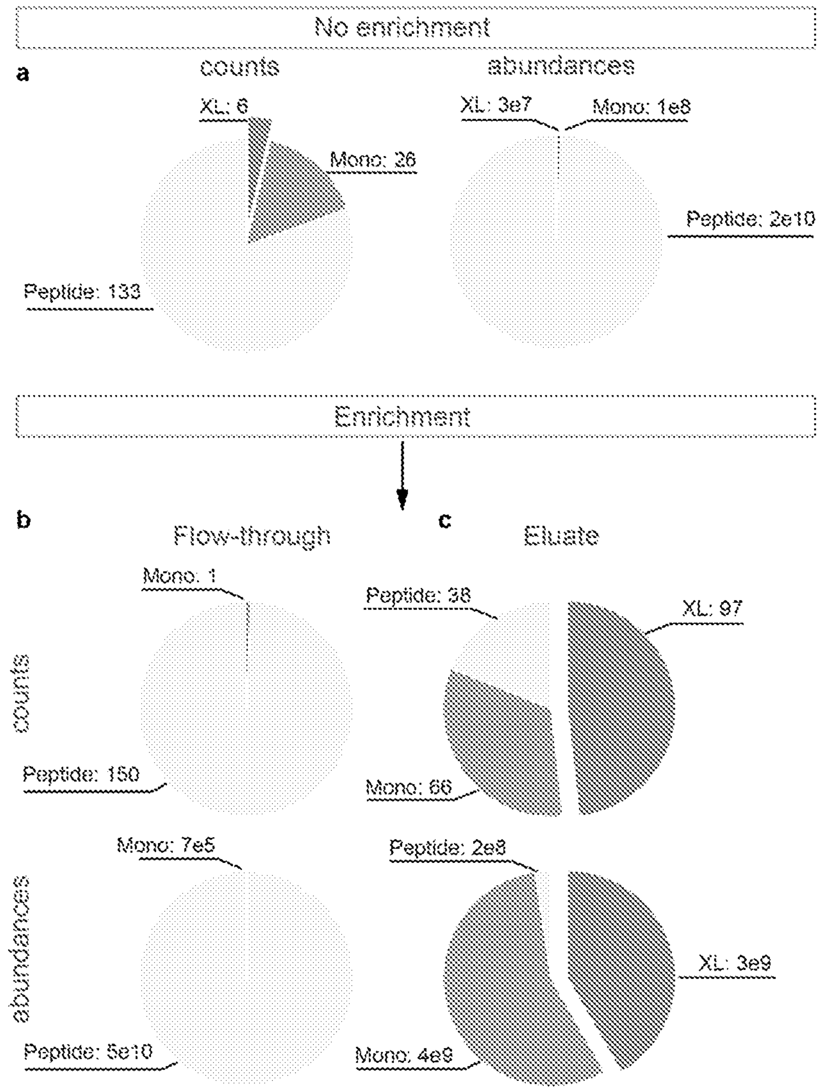
No enrichment
a
counts
abundances
XL: 6
Mono: 26
Peptide: 133
XL: 3e7
Mono: 1e8
Peptide: 2e10
Enrichment
b
Flow-through
c
Eluate
counts
Mono. 1
Peptide: 150
Peptide: 38
XL: 97
Mono: 66
abundances
Mono: 7e5
Peptide: 5e10
Peptide: 2e8
XL: 3e9
Mono: 4e9

**Figure 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120107855 A1
- US 8642744 B2
- EP 0481370 A2


### Non-patent literature cited in the description


- **D. TAN et al.** Trifunctional cross-linker for mapping proteinprotein interaction networks and comparing protein conformational states. *eLIFE,* 2016, vol. 5, e12509
- **R.M. KAAKE et al.** , A new in vivo cross-linking mass spectrometry platform to define proteinprotein interactions in living cells. *Mol. Cell. Proteomics,* 2014, vol. 13, 3533-3543
- **MARTA et al.** *Journal of Biological Chemistry,* 1996, vol. 271 (13), 7473-7478
- **H. HUANG et al.** , Simultaneous Enrichment of Cysteine containing Peptides and Phosphopeptides Using a Cysteine-specific Phosphonate Adaptable Tag (CysPAT) in Combination with titanium dioxide (TiO2) Chromatography. *Mol. Cell. Proteomics,* 2016, vol. 10, 3282-3296
- **A. BOYSEN et al.** A novel mass spectrometric strategy "REMAP" reveals Extensive O-linked protein glycosylation in Enterotoxigenic Escherichia coli. *Scientific Reports,* 2016, vol. 6, 32016
- **P.J. BOERSMA ; S. MOHAMMED ; A.J.R. HECK.** Phosphopeptide fragmentation and analysis by mass spectrometry. *J. Mass Spect.,* 2009, vol. 44, 861-878
- **A. KAO et al.** Development of a novel cross-linking strategy for fast and accurate identification of cross-linked peptides of protein complexes,. *Molecular and Cellular Proteomics,* 2011
- **MQ MÜLLER ; F. DREIOCKER ; C.H. IHLING ; M. SCHÄFER ; A. SINZ.** Cleavable cross-linker for protein structure analysis: reliable identification of cross-linking products by tandem MS,. *Analytical Chemistry,* 2010
- **A.S. ARGO ; C. SHI ; F. LIU ; M.B. GOSHE.** linking using gas-phase cleavable chemical crosslinkers and liquid chromatography-tandem mass spectrometry. *Methods,* 2015, vol. 89, 64-73